# EUROPEAN PATENT SPECIFICATION

(11) **EP 1 478 778 B1**
(45) Date of publication and mention of the grant of the patent: **13.05.2009**
(21) Application number: 03714760.0
(22) Date of filing: 27.02.2003
(51) Int. Cl.: C12Q 1/68

(54) **METHOD AND NUCLEIC ACIDS FOR THE ANALYSIS OF A COLON CELL PROLIFERACTIVE DISORDER**
VERFAHREN UND NUKLEINSÄUREN ZUR ANALYSE VON KOLONKREBSZELLEN
PROCEDE ET ACIDES NUCLEIQUES PERMETTANT L'ANALYSE D'UN TROUBLE PROLIFERATIF DES CELLULES DU COLON

(30) Priority: 27.02.2002 EP 02004551
(43) Date of publication of application: 24.11.2004
(73) Proprietor: Epigenomics AG, 10435 Berlin (DE)
(72) Inventor: ADORJAN, Peter, 10437 Berlin (DE); BURGER, Matthias, 10967 Berlin (DE); MAIER, Sabine, 12163 Berlin (DE); NIMMRICH, Inko, 10407 Berlin (DE); BECKER, Evelyne, 10437 Berlin (DE); LESCHE, Ralf, 10439 Berlin (DE); RUJAN, Tamas, 13189 Berlin (DE); SCHMITT, Armin, 96149 Breitengüssbach (DE)
(74) Representative: Krauss, Jan
(86) International application number: PCT/EP2003/002035
(87) International publication number: WO 2003/072821

(56) References cited:
- WO-A-01/07647
- WO-A-01/09183
- WO-A-03/064701
- JASS J R ET AL: "Hyperplastic polyps and DNA microsatellite unstable cancers of the colorectum" HISTOPATHOLOGY (OXFORD), vol. 37, no. 4, October 2000 (2000-10), pages 295-301, XP002363873 ISSN: 0309-0167
- LIANG G ET AL: "THE GENE FOR A NOVEL TRANSMEMBRANE PROTEIN CONTAINING EPIDERMAL GROWTH FACTOR AND FOLLISTATIN DOMAINS IS FREQUENTLY HYPERMETHYLATED IN HUMAN TUMOR CELLS" CANCER RESEARCH, AMERICAN ASSOCIATION FOR CANCER RESEARCH, BALTIMORE, MD, US, vol. 60, no. 17, 1 September 2000 (2000-09-01), pages 4907-4912, XP001197269 ISSN: 0008-5472
- YOUNG J ET AL: "HPP1: a transmembrane protein-encoding gene commonly methylated in colorectal polyps and cancers" PROCEEDINGS OF THE NATIONAL ACADEMY OF SCIENCES OF USA, NATIONAL ACADEMY OF SCIENCE, WASHINGTON, DC, US, vol. 98, no. 1, 2 January 2001 (2001-01-02), pages 265-270, XP002312905 ISSN: 0027-8424
- HILTUNEN MO ET AL: "HYPERMETHYLATION OF THE WT1 AND CALCITONIN GENE PROMOTER REGIONS ATCHROMOSOME 11P IN HUMAN COLORECTAL CANCER" BRITISH JOURNAL OF CANCER, LONDON, GB, vol. 76, no. 9, 1997, pages 1124-1130, XP000979772 ISSN: 0007-0920
- HARADA TAISHI ET AL: "Association of MDR1 expression with methylation status of 5' CPG sites at the promoter region and with mismatch repair status in colorectal tumor cell lines." PROCEEDINGS OF THE AMERICAN ASSOCIATION FOR CANCER RESEARCH ANNUAL, no. 41, March 2000 (2000-03), page 180, XP008020718 91st Annual Meeting of the American Association for Cancer Research.;San Francisco, California, USA; April 01-05, 2000, March, 2000 ISSN: 0197-016X
- GOESSL C ET AL: "DNA-Based detection of prostate cancer in blood, urine and ejaculates" ANNALS OF THE NEW YORK ACADEMY OF SCIENCES, vol. 945, no. 1, 1 September 2001 (2001-09-01), pages 51-58, XP002962894
- MODEL ET AL: "Identification and validation of colorectal neoplasia-specific methylation markers for accurate classification of disease" MOLECULAR CANCER RESEARCH, vol. 5, no. 2, pages 153-163,
- EBERT ET AL: "Hypermethylation of the TPEF/HPP1 gene in primary and metastatic colorectal cancers" NEOPLASIA, vol. 7, no. 8, August 2005 (2005-08), pages 771-778, NEW YORK
- SABBIONI ET AL: "Multigene methylation analysis of gastrointestinal tumors: TPEF emerges as a frequent tumor-specific aberrantly methylated marker that can be detected in peripheral blood." MOLECULAR DIAGNOSIS, vol. 7, no. 3-4, 2003, pages 201-207,

## Description

### Field of the Invention

The levels of observation that have been studied by the methodological developments of recent years in molecular biology, are the genes themselves, the translation of these genes into RNA, and the resulting proteins. The question of which gene is switched on at which point in the course of the development of an individual, and how the activation and inhibition of specific genes in specific cells and tissues are controlled is correlatable to the degree and character of the methylation of the genes or of the genome. In this respect, pathogenic conditions may manifest themselves in a changed methylation pattern of individual genes or of the genome.

Colorectal cancer is the fourth leading cause of cancer mortality in men and women, although ranking third in frequency in men and second in women. The 5-year survival rate is 61 % over all stages with early detection being a prerequisite for curative therapy of the disease. Up to 95% of all colorectal cancers are adenocarcinomas of varying differentiation grades.

Sporadic colon cancer develops in a multistep process starting with the pathologic transformation of normal colonic epithelium to an adenoma which consecutively progresses to invasive cancer. The progression rate of benign colonic adenomas depends strongly on their histologic appearance: whereas tubular-type adenomas tend to progress to malignant tumors very rarely, villous adenomas, particularly if larger than 2 cm in diameter, have a significant malignant potential.

During progression from benign proliferative lesions to malignant neoplasms several genetic and epigenetic alterations occur. Somatic mutation of the APC gene seems to be one of the earliest events in 75 to 80% of colorectal adenomas and carcinomas. Activation of K-RAS is thought to be a critical step in the progression towards a malignant phenotype. Consecutively, mutations in other oncogenes as well as alterations leading to inactivation of tumour suppressor genes accumulate.

Aberrant DNA methylation within CpG islands is among the earliest and most common alterations in human malignancies leading to abrogation or overexpression of a broad spectrum of genes. In addition, abnormal methylation has been shown to occur in CpG rich regulatory elements in intronic and coding parts of genes for certain tumours. In contrast to the specific hypermethylation of tumour suppressor genes, an overall hypomethylation of DNA can be observed in tumour cells. This decrease in global methylation can be detected early, far before the development of frank tumour formation. Also, correlation between hypomethylation and increased gene expression was reported for many oncogenes. In colon cancer, aberrant DNA methylation constitutes one of the most prominent alterations and inactivates many tumor suppressor genes such as p14ARF, p16INK4a, THBS1, MINT2, and MINT31 and DNA mismatch repair genes such as hMLH1.

In the molecular evolution of colorectal cancer, DNA methylation errors have been suggested to play two distinct roles. In normal colonic mucosa cells, methylation errors accumulate as a function of age or as time-dependent events predisposing these cells to neoplastic transformation. For example, hypermethylation of several loci could be shown to be already present in adenomas, particularly in the tubulovillous and villous subtype. At later stages, increased DNA methylation of CpG islands plays an important role in a subset of tumours affected by the so called CpG island methylator phenotype (CIMP). Most CIMP+ tumours, which constitute about 15% of all sporadic colorectal cancers, are characterised by microsatellite instability (MIN) due to hypermethylation of the hMLH1 promoter and other DNA mismatch repair genes. By contrast, CIMP- colon cancers evolve along a more classic genetic instability pathway (CIN), with a high rate of p53 mutations and chromosomal changes.

However, the molecular subtypes do not only show varying frequencies regarding molecular alterations. According to the presence of either micro satellite instability or chromosomal aberrations, colon cancer can be subclassified into two classes, which also exhibit significant clinical differences. Almost all MIN tumours originate in the proximal colon (ascending and transversum), whereas 70% of CIN tumours are located in the distal colon and rectum. This has been attributed to the varying prevalence of different carcinogens in different sections of the colon. Methylating carcinogens, which constitute the prevailing carcinogen in the proximal colon have been suggested to play a role in the pathogenesis of MIN cancers, whereas CIN tumours are thought to be more frequently caused by adduct-forming carcinogens, which occur more frequently in distal parts of the colon and rectum. Moreover, MIN tumours have a better prognosis than do tumours with a CIN phenotype and respond better to adjuvant chemotherapy.

The identification of markers for the differentiation of colon carcinoma as well as for early detection are main goals of current research.

5-methylcytosine is the most frequent covalent base modification in the DNA of eukaryotic cells. It plays a role, for example, in the regulation of the transcription, in genetic imprinting, and in tumorigenesis. Therefore, the identification of 5-methylcytosine as a component of genetic information is of considerable interest. However, 5-methylcytosine positions cannot be identified by sequencing since 5-methylcytosine has the same base pairing behaviour as cytosine. Moreover, the epigenetic information carried by 5-methylcytosine is completely lost during PCR amplification.

A relatively new and currently the most frequently used method for analysing DNA for 5-methylcytosine is based upon the specific reaction of bisulfite with cytosine which, upon subsequent alkaline hydrolysis, is converted to uracil which corresponds to thymidine in its base pairing behaviour. However, 5-methylcytosine remains unmodified under these conditions. Consequently, the original DNA is converted in such a manner that methylcytosine, which originally could not be distinguished from cytosine by its hybridisation behaviour, can now be detected as the only remaining cytosine using "normal" molecular biological techniques, for example, by amplification and hybridisation or sequencing. All of these techniques are based on base pairing which can now be fully exploited. In terms of sensitivity, the prior art is defined by a method which encloses the DNA to be analysed in an agarose matrix, thus preventing the diffusion and renaturation of the DNA (bisulfite only reacts with single-stranded DNA), and which replaces all precipitation and purification steps with fast dialysis (Olek A, Oswald J, Walter J. A modified and improved method for bisulphite based cytosine methylation analysis. Nucleic Acids Res. 1996 Dec 15;24(24):5064-6). Using this method, it is possible to analyse individual cells, which illustrates the potential of the method. However, currently only individual regions of a length of up to approximately 3000 base pairs are analysed, a global analysis of cells for thousands of possible methylation events is not possible. However, this method cannot reliably analyse very small fragments from small sample quantities either. These are lost through the matrix in spite of the diffusion protection.

An overview of the further known methods of detecting 5-methylcytosine may be gathered from the following review article: Rein, T., DePamphilis, M. L., Zorbas, H., Nucleic Acids Res.1998,26,2255.

To date, barring few exceptions (e.g., Zeschnigk M, Lich C, Buiting K, Doerfler W, Horsthemke B. A single-tube PCR test for the diagnosis of Angelman and Prader-Willi syndrome based on allelic methylation differences at the SNRPN locus. Eur J Hum Genet. 1997 Mar-Apr;5(2):94-8) the bisulfite technique is only used in research. Always, however, short, specific fragments of a known gene are amplified subsequent to a bisulfite treatment and either completely sequenced (Olek A, Walter J. The pre-implantation ontogeny of the H19 methylation imprint. Nat Genet. 1997 Nov;17(3):275-6) or individual cytosine positions are detected by a primer extension reaction (Gonzalgo ML, Jones PA. Rapid quantitation of methylation differences at specific sites using methylation-sensitive single nucleotide primer extension (Ms-SNuPE). Nucleic Acids Res. 1997 Jun 15;25(12):2529-31, WO 95/00669) or by enzymatic digestion (Xiong Z, Laird PW. COBRA: a sensitive and quantitative DNA methylation assay. Nucleic Acids Res. 1997 Jun 15;25(12):2532-4). In addition, detection by hybridisation has also been described (Olek et al., WO 99/28498).

Further publications dealing with the use of the bisulfite technique for methylation detection in individual genes are: Grigg G, Clark S. Sequencing 5-methylcytosine residues in genomic DNA. Bioessays. 1994 Jun;16(6):431-6, 431; Zeschnigk M, Schmitz B, Dittrich B, Buiting K, Horsthemke B, Doerfler W. Imprinted segments in the human genome: different DNA methylation patterns in the Prader-Willi/Angelman syndrome region as determined by the genomic sequencing method. Hum Mol Genet. 1997 Mar;6(3):387-95; Feil R, Charlton J, Bird AP, Walter J, Reik W. Methylation analysis on individual chromosomes: improved protocol for bisulphite genomic sequencing. Nucleic Acids Res. 1994 Feb 25;22(4):695-6; Martin V, Ribieras S, Song-Wang X, Rio MC, Dante R. Genomic sequencing indicates a correlation between DNA hypomethylation in the 5' region of the pS2 gene and its expression in human breast cancer cell lines. Gene. 1995 May 19;157(1-2):261-4; WO 97/46705 and WO 95/15373.

An overview of the Prior Art in oligomer array manufacturing can be gathered from a special edition of Nature Genetics (Nature Genetics Supplement, Volume 21, January 1999), published in January 1999, and from the literature cited therein.

Fluorescently labelled probes are often used for the scanning of immobilised DNA arrays. The simple attachment of Cy3 and Cy5 dyes to the 5'-OH of the specific probe are particularly suitable for fluorescence labels. The detection of the fluorescence of the hybridised probes may be carried out, for example via a confocal microscope. Cy3 and Cy5 dyes, besides many others, are commercially available.

Matrix Assisted Laser Desorption Ionization Mass Spectrometry (MALDI-TOF) is a very efficient development for the analysis of biomolecules (Karas M, Hillenkamp F. Laser desorption ionisation of proteins with molecular masses exceeding 10,000 daltons. Anal Chem. 1988 Oct 15;60(20):2299-301). An analyte is embedded in a light-absorbing matrix. The matrix is evaporated by a short laser pulse thus transporting the analyte molecule into the vapour phase in an unfragmented manner. The analyte is ionised by collisions with matrix molecules. An applied voltage accelerates the ions into a field-free flight tube. Due to their different masses, the ions are accelerated at different rates. Smaller ions reach the detector sooner than bigger ones.

MALDI-TOF spectrometry is excellently suited to the analysis of peptides and proteins. The analysis of nucleic acids is somewhat more difficult (Gut I G, Beck S. DNA and Matrix Assisted Laser Desorption Ionisation Mass Spectrometry. Current Innovations and Future Trends. 1995, 1; 147-57). The sensitivity to nucleic acids is approximately 100 times worse than to peptides and decreases disproportionally with increasing fragment size. For nucleic acids having a multiply negatively charged backbone, the ionisation process via the matrix is considerably less efficient. In MALDI-TOF spectrometry, the selection of the matrix plays an eminently important role. For the desorption of peptides, several very efficient matrixes have been found which produce a very fine crystallisation. There are now several responsive matrixes for DNA, however, the difference in sensitivity has not been reduced. The difference in sensitivity can be reduced by chemically modifying the DNA in such a manner that it becomes more similar to a peptide. Phosphorothioate nucleic acids in which the usual phosphates of the backbone are substituted with thiophosphates can be converted into a charge-neutral DNA using simple alkylation chemistry (Gut IG, Beck S. A procedure for selective DNA alkylation and detection by mass spectrometry. Nucleic Acids Res. 1995 Apr 25;23(8):1367-73). The coupling of a charge tag to this modified DNA results in an increase in sensitivity to the same level as that found for peptides. A further advantage of charge tagging is the increased stability of the analysis against impurities which make the detection of unmodified substrates considerably more difficult.

Genomic DNA is obtained from DNA of cell, tissue or other test samples using standard methods. This standard methodology is found in references such as Sambrook, Fritsch and Maniatis eds., Molecular Cloning: A Laboratory Manual, 1989.

Young et al. (in Young J et al. HPP1: a transmembrane protein-encoding gene commonly methylated in colorectal polyps and cancers. J Proc Natl Acad Sci U S A. 2001, Jan 2:98(1):265-70) describe the use of HPP1 (TPEF) methylation in various colon cell proliferative disorders, wherein methylation is determined as characteristic of neoplastic evolution.

Goessl et al. (in Goessl et al, DNA-based detection of prostate cancer in blood, urine an ejaculates. Annals of the New York Academy of Sciences, vol 945, No. 1, September 2001, pp 51-58) describe the use of the GSTP1 as a methylation marker to detect prostate cancer in blood, urine an ejaculates.

### Description

The invention provides a method for detecting and differentiating between colon cell proliferative disorders, comprising the following steps ; a) providing a body fluid sample containing genomic DNA, b) extracting said genomic DNA, c) converting cytosine bases in said genomic DNA sample which are unmethylated at the 5-position, by treatment, to uracil or another base which is dissimilar to cytosine in terms of base pairing behaviour, and d) identifying the methylation status of one or more cytosine positions of the gene TPEF and/or its regulatory regions.

The present invention makes available a method for ascertaining genetic and/or epigenetic parameters of genomic DNA. The method is for use in the improved diagnosis, treatment and monitoring of colon cell proliferative disorders, more specifically by enabling the improved identification of and differentiation between subclasses of said disorder and the genetic predisposition to said disorders. The invention presents improvements over the state of the art in that it enables a highly specific classification of colon carcinomas, thereby allowing for improved and informed treatment of patients.

In a particularly preferred embodiment the present invention makes available methods that allow the differentiation between colon carcinoma, colon adenoma and normal colon tissue.

Furthermore, the method enables the analysis of cytosine methylations and single nucleotide polymorphisms.

The gene that forms the basis of the present invention can be used to form a "gene panel", i.e. a collection comprising the particular genetic sequences of the present invention and/or their respective informative methylation sites. The formation of gene panels allow for a quick and specific analysis of the disorders they are related with. The gene panels described in this invention can be used with surprisingly high efficiency for the diagnosis, treatment and monitoring of and the analysis of colon cell proliferative disorders as described herein. The use of multiple CpG sites from a diverse array of genes, allows for a relatively high degree of sensitivity and specificity in comparison to single gene diagnostic and detection tools, Furthermore, the panel as described herein may be adapted for use in the analysis of many aspects of colon cell proliferative disorders.

In a preferred embodiment, the method comprises the following steps:

In the first step of the method the genomic DNA sample must be isolated from body fluids. Extraction may be by means that are standard to one skilled in the art, these include the use of detergent lysates, sonification and vortexing with glass beads. Once the nucleic acids have been extracted the genomic double stranded DNA is used in the analysis.

In a preferred embodiment the DNA may be cleaved prior to the next step of the method, this may be by any means standard in the state of the art, in particular, but not limited to, with restriction endonucleases.

In the second step of the method, the genomic DNA sample is treated in such a manner that cytosine bases which are unmethylated at the 5'-position are converted to uracil, thymine, or another base which is dissimilar to cytosine in terms of hybridisation behaviour. This will be understood as 'pretreatment' hereinafter.

The above described treatment of genomic DNA is preferably carried out with bisulfite (sulfite, disulfite) and subsequent alkaline hydrolysis which results in a conversion of non-methylated cytosine nucleobases to uracil or to another base which is dissimilar to cytosine in terms of base pairing behaviour. If bisulfite solution is used for the reaction, then an addition takes place at the non-methylated cytosine bases. Moreover, a denaturating reagent or solvent as well as a radical interceptor must be present. A subsequent alkaline hydrolysis then gives rise to the conversion of non-methylated cytosine nucleobases to uracil. The chemically converted DNA is then used for the detection of methylated cytosines.

Fragments of the pretreated DNA are amplified, using sets of primer oligonucleotides accordine to SEQ ID NO: 239, 240, 367, and 368, and a preferably heat-stable, polymerase. Because of statistical and practical considerations, preferably more than ten different fragments having a length of 100 - 2000 base pairs are amplified. The amplification of several DNA segments can be carried out simultaneously in one and the same reaction vessel. Usually, the amplification is carried out by means of a polymerase chain reaction (PCR).

The method may also be enabled by the use of alternative primers, the design of such primers is obvious to one skilled in the art. These should include at least two oligonucleotides whose sequences are each reverse complementary or identical to an at least 18 base-pair long segment of the base sequences specified in the appendix (SEQ ID NO: 239, 240, 367, and 368). Said primer oligonucleotides are preferably characterised in that they do not contain any CpG dinucleotides. In a particularly preferred embodiment of the method, the sequence of said primer oligonucleotides are designed so as to selectively anneal to and amplify, only the colon tissue specific DNA of interest, thereby minimising the amplification of background or non relevant DNA. In the context of the present invention, background DNA is taken to mean genomic DNA which does not have a relevant tissue specific methylation pattern, in this case, the relevant tissue being colon, both healthy and diseased.

According to the present invention, it is preferred that at least one primer oligonucleotide is bound to a solid phase during amplification. The different oligonucleotide and/or PNA-oligomer sequences can be arranged on a plane solid phase in the form of a rectangular or hexagonal lattice, the solid phase surface preferably being composed of silicon, glass, polystyrene, aluminium, steel, iron, copper, nickel, silver, or gold, it being possible for other materials such as nitrocellulose or plastics to be used as well.

The fragments obtained by means of the amplification can carry a directly or indirectly detectable label. Preferred are labels in the form of fluorescence labels, radionuclides, or detachable molecule fragments having a typical mass which can be detected in a mass spectrometer, it being preferred that the fragments that are produced have a single positive or negative net charge for better detectability in the mass spectrometer. The detection may be carried out and visualised by means of matrix assisted laser desorption/ionisation mass spectrometry (MALDI) or using electron spray mass spectrometry (ESI).

The amplificates obtained in the second step of the method are subsequently hybridised to an array or a set of oligonucleotides and/or PNA probes. In this context, the hybridisation preferably takes place in the manner described as follows. The set of probes used during the hybridisation is preferably composed of at least 10 oligonucleotides or PNA-oligomers. However, it is understood and as well claimed, that the process can be conducted using only one Oligonucleotide or PNA probe. In the process, the amplificates hybridise to oligonucleotides previously bonded to a solid phase. The oligonucleotides can be taken from the group comprising SEQ ID NO: 65 to SEQ ID NO: 132, and SEQ ID NO: 519 to SEQ ID NO: 1030. The oligonucleotides can also be taken from the group comprising SEQ ID NO: 895 to SEQ ID NO: 1030. The non-hybridised fragments are subsequently removed. Said oligonucleotides contain at least one base sequence having a length of 10 nucleotides which is reverse complementary or identical to a segment of the base sequences specified in the appendix, the segment containing at least one CpG or TpG dinucleotide. In a further preferred embodiment the cytosine of the CpG dinucleotide, or in the case of TpG, the thiamine, is the 5^{th} to 9^{th} nucleotide from the 5'-end of the 10-mer. One oligonucleotide exists for each CpG or TpG dinucleotide.

In the fifth step of the method, the non-hybridised amplificates are removed.

In the final step of the method, the hybridised amplificates are detected. In this context, it is preferred that labels attached to the amplificates are identifiable at each position of the solid phase at which an oligonucleotide sequence is located.

According to the present invention, it is preferred that the labels of the amplificates are fluorescence labels, radionuclides, or detachable molecule fragments having a typical mass which can be detected in a mass spectrometer. The mass spectrometer is preferred for the detection of the amplificates, fragments of the amplificates or of probes which are complementary to the amplificates, it being possible for the detection to be carried out and visualised by means of matrix assisted laser desorption/ionisation mass spectrometry (MALDI) or using electron spray mass spectrometry (ESI). The produced fragments may have a single positive or negative net charge for better detectability in the mass spectrometer.

The aforementioned method is preferably used for ascertaining genetic and/or epigenetic parameters of genomic DNA.

In order to enable this method, the invention further describes the modified DNA of genes MDR1, APOC2, CACNA1G, EGR4, AR, RB1, GPIb beta, MYOD1, WT1, HLA-F, ELK1, APC, BCL2, CALCA, CDH1, CDKN1A, CDKN1B (p27 Kip1), CDKN2a, CDKN2B, CD44, CSPG2, DARK1, EGFR, EYA4, GSTP1, GTBP/MSH6, HIC-1, HRAS, IGF2, LKB1, MGMT, MLH1, MNCA9, MSH3, MYC, N33, PAX6, PGR, PTEN, RARB, SFN, S100A2, TGFBR22, TIMP3, TP53, TP73, VHL, CDKN1C, CAV1, CDH13, DRG1, PTGS2, THBS1, TPEF (=TMEFF2; =HPP1), DNMT1, CEA, MB, PCNA, CDC2 , ESR1, CASP8, RASSF1, MSH4, MSH5 as well as oligonucleotides and/or PNA-oligomers for detecting cytosine methylations within said genes. The present invention is based on the discovery that genetic and epigenetic parameters and, in particular, the cytosine methylation patterns of genomic DNA are particularly suitable for improved diagnosis, treatment and monitoring of colon cell proliferative disorders. Furthermore, the invention enables the differentiation between different subclasses of colon cell proliferative disorders or detection of a predisposition to colon cell proliferative disorders,

The nucleic acids as described can be used for the analysis of genetic and/or epigenetic parameters of genomic DNA.

This objective is achieved according to the present invention using a nucleic acid containing a sequence of at least 18 bases in length of the pretreated genomic DNA according to one of SEQ ID NO: 239, 240, 367 and 368 and sequences complementary thereto.

The modified nucleic acid could heretofore not be connected with the ascertainment of disease relevant genetic and epigenetic parameters.

The present invention further describes an oligonucleotide or oligomer for the analysis of pretreated DNA, for detecting the genomic cytosine methylation state, said oligonucleotide containing at least one base sequence having a length of at least 10 nucleotides which hybridises to a pretreated genomic DNA according to SEQ ID NO: 133 through to SEQ ID NO: 388. The oligomer probes constitute important and effective tools which, for the first time, make it possible to ascertain specific genetic and epigenetic parameters during the analysis of biological samples for features associated with the development of colon cell proliferative disorders. Said oligonucleotides allow the improved diagnosis, treatment and monitoring of colon cell proliferative disorders and detection of the predisposition to said disorders. Furthermore, they allow the differentiation of different subclasses of colon cell proliferative disorders. The base sequence of the oligomers preferably contains at least one CpG or TpG dinucleotide. The probes may also exist in the form of a PNA (peptide nucleic acid) which has particularly preferred pairing properties. Particularly preferred are oligonucleotides according to the present invention in which the cytosine of the CpG dinucleotide is the 5^{th} - 9^{th} nucleotide from the 5'-end of the 13-mer; in the case of PNA-oligomers, it is preferred for the cytosine of the CpG dinucleotide to be the 4^{th} - 6^{th} nucleotide from the 5'-end of the 9-mer.

The oligomers according to the present invention are normally used in so called "sets" which contain at least one oligomer for each of the CpG dinucleotides within SEQ_ID NO: 239, 240, 367, and 368. Preferred is a set which contains at least one oligomer for each of the CpG dinucleotides, selected from SEQ ID NO: 65 to SEQ ID NO: 132, and SEQ ID NO: 519 to SEQ ID NO: 1030. Further preferred is a selection from the set comprising SEQ ID NO: 895 to SEQ ID NO: 1030.

In the case of the sets of oligonucleotides according to the present invention, it is preferred that at least one oligonucleotide is bound to a solid phase. It is further preferred that all the oligonucleotides of one set are bound to a solid phase.

The present invention moreover relates to a set of preferably at least 10 n (oligonucleotides and/or PNA-oligomers) used for detecting the cytosine methylation state of genomic DNA using treated versions of said genomic DNA (according to SEQ ID NO: 239, 240, 367, and 368 and sequences complementary thereto). However, it is understood and as well claimed, that the process can be conducted using only one Oligonucleotide or PNA oligomer. These probes enable improved diagnosis, treatment and monitoring of colon cell proliferative disorders. In particular they enable the differentiation between different sub classes of colon cell proliferative disorders and the detection of a predisposition to said disorders. In a particularly preferred embodiment the set comprises SEQ ID NO: 65 to SEQ ID NO: 132, and SEQ ID NO: 519 to SEQ ID NO: 1030.

The set of oligomers may also be used for detecting single nucleotide polymorphisms (SNPs) using pretreated genomic DNA according to one of SEQ ID NO: 239, 240, 367, and 368.

Described herein is an arrangement of different oligonucleotides and/or PNA-oligomers (a socalled "array") present in a manner that it is likewise bound to a solid phase. This array of different oligonucleotide- and/or PNA-oligomer sequences can be characterised in that it is arranged on the solid phase in the form of a rectangular or hexagonal lattice. The solid phase surface is preferably composed of silicon, glass, polystyrene, aluminium, steel, iron, copper, nickel, silver, or gold. However, nitrocellulose as well as plastics such as nylon which can exist in the form of pellets or also as resin matrices are suitable alternatives.

Described herein is a method for manufacturing an array fixed to a carrier material for the improved diagnosis, treatment and monitoring of colon cell proliferative disorders, the differentiation between different subclasses of colon cell proliferative disorders and/or detection of the predisposition to colon cell proliferative disorders. In said method, at least one oligomer as described is coupled to a solid phase. Methods for manufacturing such arrays are known, for example, from Patent US 5,744,305 by means of solid-phase chemistry and photolabile protecting groups.

Described herein is a DNA chip for the improved diagnosis, treatment and monitoring of colon cell proliferative disorders, Furthermore the DNA chip enables detection of the predisposition to colon cell proliferative disorders and the differentiation between different subclasses of colon cell proliferative disorders. The DNA chip contains at least one nucleic acid as described. DNA chips are known, for example, in Patent US 5,837,832.

Moreover, described herein is a kit which may be composed, for example, of a bisulfite-containing reagent, a set of primer oligonucleotides containing at least two oligonucleotides whose sequences in each case correspond or are complementary to a 18 base long segment of the base sequences specified in the appendix (SEQ ID NO: 133 to SEQ ID NO: 388), oligonucleotides and/or PNA-oligomers as well as instructions for carrying out and evaluating the described method. However, a kit as described can also contain only part of the aforementioned components.

The oligomers or arrays thereof as well as a kit as described are intended to be used for the improved diagnosis, treatment and monitoring of colon cell proliferative disorders. Furthermore the use of said inventions extends to the differentiation between different subclasses of colon cell proliferative disorders and detection of the predisposition to colon cell proliferative disorders. According to the present invention, the method is preferably used for the analysis of important genetic and/or epigenetic parameters within genomic DNA, in particular for use in improved diagnosis, treatment and monitoring of colon cell proliferative disorders, detection of the predisposition to said disorders and the differentiation between subclasses of said disorders.

The methods according to the present invention are used, for example, for improved diagnosis, treatment and monitoring of colon cell proliferative disorders progression, detection of the predisposition to said disorders and the differentiation between subclasses of said disorders.

A further embodiment of the invention is a method for the analysis of the methylation status of genomic DNA of the gene TPEF without the need for pretreatment. In the first step of the method the genomic DNA sample must be isolated from body fluids. Extraction may be by means that are standard to one skilled in the art, these include the use of detergent lysates, sonification and vortexing with glass beads. Once the nucleic acids have been extracted the genomic double stranded DNA is used in the analysis.

In a preferred embodiment the DNA may be cleaved prior to the treatment, this may be any means standard in the state of the art, in particular with restriction endonucleases, In the second step, the DNA is then digested with one or more methylation sensitive restriction enzymes. The digestion is carried out such that hydrolysis of the DNA at the restriction site is informative of the methylation status of a specific CpG dinucleotide.

In the third step the restriction fragments are amplified. In a preferred embodiment this is carried out using a polymerase chain reaction.

In the final step the amplificates are detected. The detection may be by any means standard in the art, for example, but not limited to, gel electrophoresis analysis, hybridisation analysis, incorporation of detectable tags within the PCR products, DNA array analysis, MALDI or ESI analysis.

The present invention moreover relates to the diagnosis and/or prognosis of events which are disadvantageous or relevant to patients or individuals in which important genetic and/or epigenetic parameters within genomic DNA, said parameters obtained by means of the present invention may be compared to another set of genetic and/or epigenetic parameters, the differences serving as the basis for the diagnosis and/or prognosis of events which are disadvantageous or relevant to patients or individuals.

In the context of the present invention the term "hybridisation" is to be understood as a bond of an oligonucleotide to a completely complementary sequence along the lines of the Watson-Crick base pairings in the sample DNA, forming a duplex structure.

In the context of the present invention, "genetic parameters" are mutations and polymorphisms of genomic DNA and sequences further required for their regulation. To be designated as mutations are, in particular, insertions, deletions, point mutations, inversions and polymorphisms and, particularly preferred, SNPs (single nucleotide polymorphism).

In the context of the present invention "methylation state analysis" is taken to mean the analysis of cytosines within a nucleic acid in order to ascertain whether they are methylated or not. In the context of the present invention, "epigenetic parameters" are, in particular, cytosine methylations and further modifications of DNA bases of genomic PNA and sequences further required for their regulation. Further epigenetic parameters include, for example, the acetylation of histones which, cannot be directly analysed using the described method but which, in turn, correlates with the DNA methylation.

In the following, the present invention will be explained in greater detail on the basis of the sequences and examples without being limited thereto.

SEQ ID NO: 1 to SEQ ID NO: 64 represent 5' and/or regulatory regions of the genomic DNA of genes MDR1, APOC2, CACNA1G, EGR4, AR, RB1, GPIb beta, MYOD1, WT1, HLA-F, ELK1, APC, BCL2, CALCA, CDH1, CDKN1A, CDKN1B (p27 Kip1), CDKN2a, CDKN2B, CD44, CSPG2, DARK1, EGFR, EYA4, GSTP1, GTBP/MSH6, HIC-1, HRAS, IGF2, LKB1, MGMT, MLH1, MNCA9, MSH3, MYC, N33, PAX6, PGR, PTEN, RARB, SFN, S100A2, TGFBR2, TIMP3, TP53, TP73, VHL, CDKN1C, CAV1, CDH13, DRG1, PTGS2, THBS1, TPEF (=TMEFF2; =HPP1), DNMT1, CEA, MB, PCNA, CDC2, ESR1, CASP8, RASSF1, MSH4, MSH5. These sequences are derived from the ensembl database (date 01.10.2001) (http://www.ensembl.org) and will be taken to include all minor variations of the sequence material which are currently unforeseen, for example, but not limited to, minor deletions and SNPs.

SEQ ID 133 to 388 exhibit the pretreated sequences of DNA derived from genes MDR1, APOC2, CACNA1G, EGR4, AR, RB1, GPIb beta, MYOD1, WT1, HLA-F, ELK1, APC, BCL2, CALCA, CDH1, CDKN1A, CDKN1B (p27 Kip1), CDKN2a, CDKN2B, CD44, CSPG2, DAPK1, EGFR, EYA4, GSTP1, GTBP/MSH6, HIC-1, HRAS, IGF2, LKB1, MGMT, MLH1, MNCA9, MSH3, MYC, N33, PAX6, PGR, PTEN, RARB, SFN, S100A2, TGFBR2, TIMP3, TP53, TP73, VHL, CDKN1C, CAV1, CDH13, DRG1, PTGS2, THBS1, TPEF (=TMEFF2; =HPP1), DNMT1, CEA, MB, PCNA, CDC2, ESR1, CASP8, RASSF1, MSH4, MSH5. These sequences will be taken to include all minor variations of the sequence material which are currently unforeseen, for example, but not limited to, minor deletions and SNPs.

SEQ ID NO: 389 to SEQ ID NO: 518 exhibit the sequences of primer oligonucleotides for the amplification of pretreated DNA according to Sequence ID NO: 133 to SEQ ID NO: 388.

SEQ ID NO: 65 to SEQ ID NO: 132 exhibit the sequences of oligomers which are useful for the analysis of CpG positions within genomic DNA according to SEQ ID NO: 1 to SEQ ID NO: 64.

SEQ ID NO: 519 to SEQ ID NO: 1030 exhibit the sequences of oligomers which are useful for the analysis of the methylation status of CpG positions within genomic DNA according to SEQ ID NO: 1 to SEQ ID NO: 64 after treatment of said genomic DNA with bisulfite.

SEQ ID NO: 895 to SEQ ID NO: 1030 exhibit the sequences of oligomers which are particularly useful for the analysis of CpG positions within genomic DNA according to SEQ ID NO: 1 to SEQ ID NO: 64, after treatment of said with bisulfite and are subject to a preferred embodiment of this invention.

### Description of figures

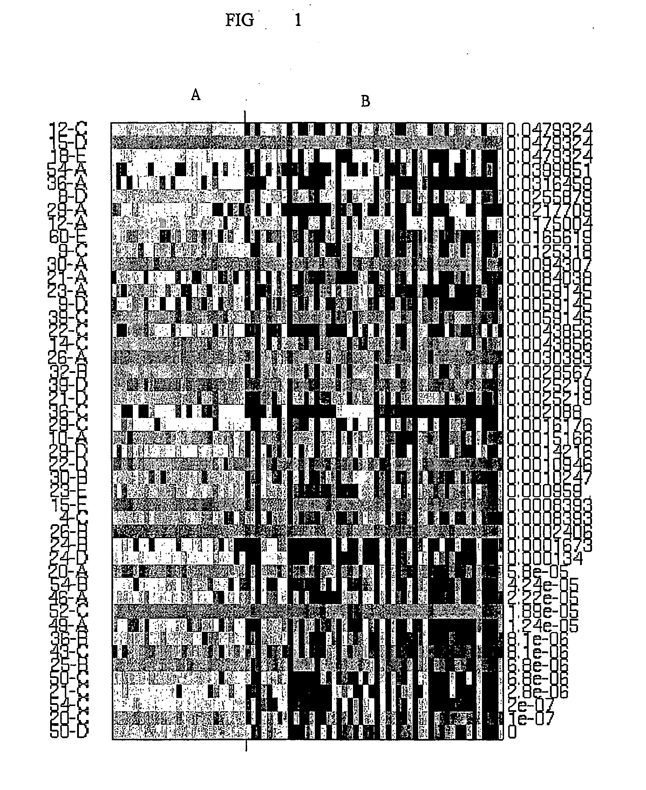

Figure 1: Differentiation between healthy colon tissue and adenoma or carcinoma colon tissue according to Example 2. The labels on the left side of the plot are gene and CpG identifiers, these can be cross referenced using Table 3 and Table 7. The labels on the right side of the figure give the significance (p-value, T-test) of the difference between the means of the two groups. Each row corresponds to a single CpG and each column to the methylation levels of one sample. CpGs are ordered according to their contribution to the differentiation between the two tissue types (A = healthy, B = non healthy) with increasing contribution from top to bottom. Black indicates total methylation at a given CpG position, white represents no methylation at the particular position, with degrees of methylation represented in grey, from light (low proportion of methylation) to dark (high proportion of methylation).

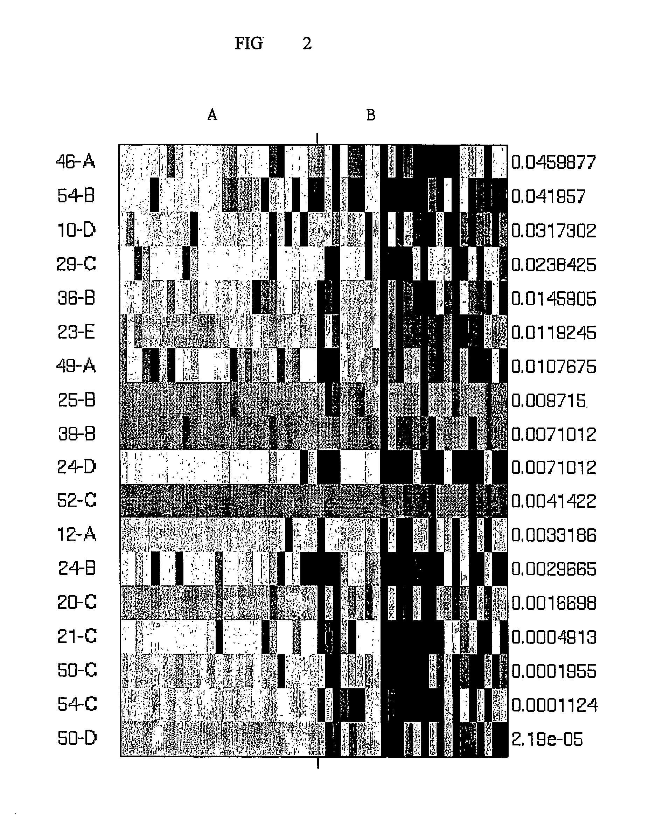

Figure 2: Differentiation between healthy colon tissue and carcinoma colon tissue according to Example 2. The labels on the left side of the plot are gene and CpG identifiers, these can be cross referenced using Table 4 and Table 7. The labels on the right side of the figure give the significance (p-value, T-test) of the difference between the means of the two groups. Each row corresponds to a single CpG and each column to the methylation levels of one sample. CpGs are ordered according to their contribution to the differentiation between the two tissue types (A = healthy, B = carcinoma) with increasing contribution from top to bottom. Black indicates total methylation at a given CpG position, white represents no methylation at the particular position, with degrees of methylation represented in grey, from light (low proportion of methylation) to dark (high proportion of methylation).

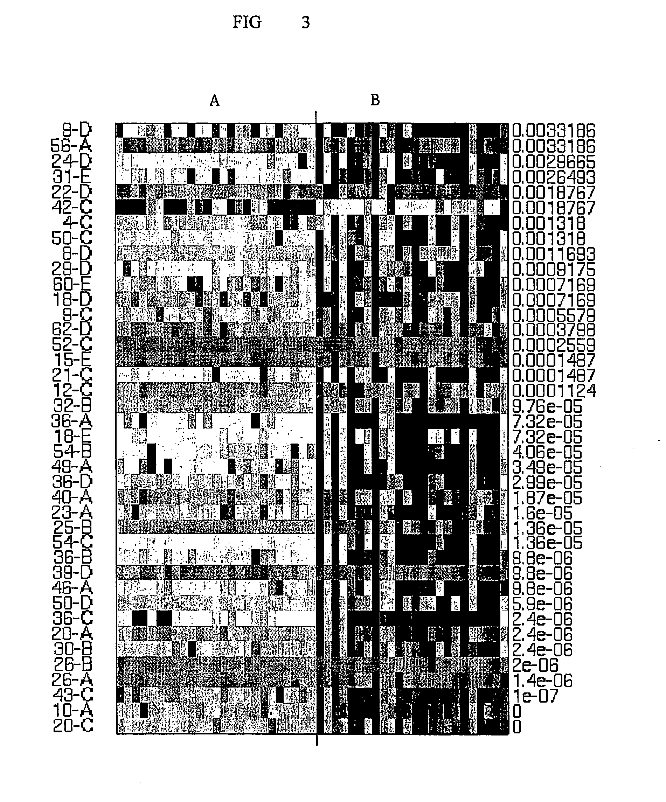

Figure 3: Differentiation between healthy colon tissue and adenoma colon tissue according to Example 2. The labels on the left side of the plot are gene and CpG identifiers, these can be cross referenced in Table 5 and Table 7. The labels on the right side give the significance (p-value, T-test) of the difference between the means of the two groups. Each row corresponds to a single CpG and each column to the methylation levels of one sample. CpGs are ordered according to their contribution to the distinction to the differential diagnosis between the two tissue types (A = healthy, B = adenoma) with increasing contribution from top to bottom. Black indicates total methylation at a given CpG position, white represents no methylation at the particular position, with degrees of methylation represented in grey, from light (low proportion of methylation) to dark (high proportion of methylation). Due to formatting of the page only 40 CpGs are shown in this figure.

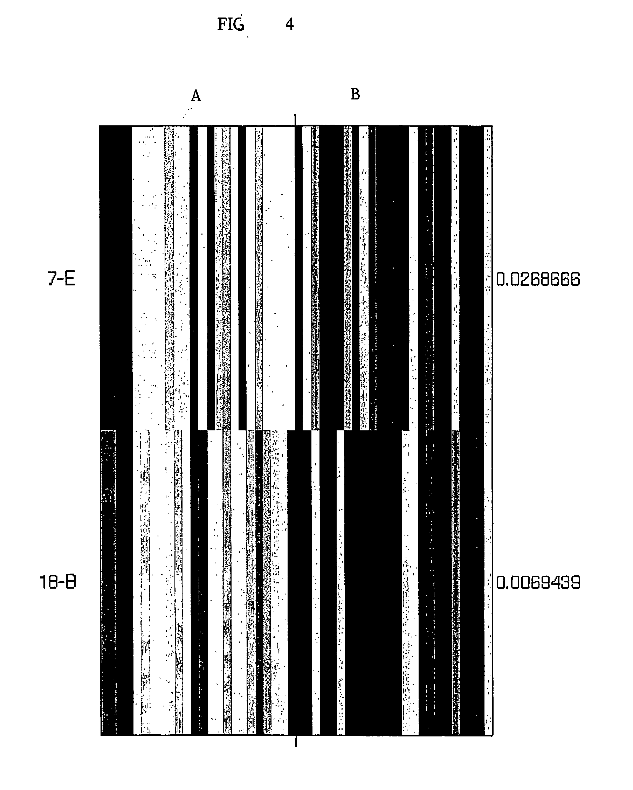

Figure 4: Differentiation between carcinoma colon tissue and adenoma colon tissue according to Example 2. The labels on the left side of the plot are gene and CpG identifiers, these can be cross referenced in Table 6 and Table 7. The labels on the right side give the significance (p-value, T-test) of the difference between the means of the two groups. Each row corresponds to a single CpG and each column to the methylation levels of one sample. CpGs are ordered according to their contribution to the distinction to the differential diagnosis between the two tissue types (A = carcinoma, B = adenoma) with increasing contribution from top to bottom. Black indicates total methylation at a given CpG position, white represents no methylation at the particular position, with degrees of methylation represented in grey, from light (low proportion of methylation) to dark (high proportion of methylation).

### Examples 1 and 2: Digital Phenotype

In the following examples, multiplex PCR was carried out upon tissue samples originating from colon adenomas or colon carcinoma. Multiplex PCR was also carried out upon healthy colon tissue. Each sample was treated in the manner described below in Example 1 in order to deduce the methylation status of CpG positions, the CpG methylation information for each sample was collated and then used in an analysis, as detailed in Example 2. An alternative method for the analysis of CpG methylation status is described in Example 3.

### Example 1

In the first step the genomic DNA was isolated from the cell samples using the Wizzard kit from (Promega).

The isolated genomic DNA from the samples are treated using a bisulfite solution (hydrogen sulfite, disulfite). The treatment is such that all non methylated cytosines within the sample are converted to thiamidine, conversely 5-methylated cytosines within the sample remain unmodified.

The treated nucleic acids were then amplified using multiplex PCRs, amplifying 8 fragments per reaction with Cy5 fluorescently labelled primers. PCR primers used are described in Table 1. PCR conditions were as follows.

### Reaction solution:

10 ng bisulfite treated DNA
3,5 mM MgCl2
400 µM dNTPs
2 pmol each primer
1 U Hot Star Taq (Qiagen)

Forty cycles were carried out as follows. Denaturation at 95°C for 15 min, followed by annealing at 55°C for 45 sec., primer elongation at 65°C for 2 min. A final elongation at 65°C was carried out for 10 min.

All PCR products from each individual sample were then hybridised to glass slides carrying a pair of immobilised oligonucleotides for each CpG position under analysis. Each of these detection oligonucleotides was designed to hybridise to the bisulphite converted sequence around one CpG site which was either originally unmethylated (TG) or methylated (CG). See Table 2 for further details of all hybridisation oligonucleotides used (both informative and non-informative) Hybridisation conditions were selected to allow the detection of the single nucleotide differences between the TG and CG variants.

5 µl volume of each multiplex PCR product was diluted in 10 x Ssarc buffer (10 x Ssarc:230 ml 20 x SSC, 180 ml sodium lauroyl sarcosinate solution 20%, dilute to 1000 ml with dH2O). The reaction mixture was then hybridised to the detection oligonucleotides as follows. Denaturation at 95°C, cooling down to 10 °C, hybridisation at 42°C overnight followed by washing with 10 x Ssarc and dH2O at 42°C.

Fluorescent signals from each hybridised oligonucleotide were detected using genepix scanner and software. Ratios for the two signals (from the CG oligonucleotide and the TG oligonucleotide used to analyse each CpG position) were calculated based on comparison of intensity of the fluorescent signals.

### Example 2

The data obtained according to Example 1 is then sorted into a ranked matrix (as shown in Figures 1 to 4) according to CpG methylation differences between the two classes of tissues, using an algorithm. The most significant CpG positions are at the bottom of the matrix with significance decreasing towards the top. Black indicates total methylation at a given CpG position, white represents no methylation at the particular position, with degrees of methylation represented in grey, from light (low proportion of methylation) to dark (high proportion of methylation). Each row represents one specific CpG position within a gene and each column shows the methylation profile for the different CpGs for one sample. On the left side a CpG and gene identifier is shown this may be cross referenced with the accompanying tables (Table 1 and 7) in order to ascertain the gene in question and the detection oligomer used. On the right side p values for the individual CpG positions are shown. The p values are the probabilities that the observed distribution occurred by chance in the data set.

For selected distinctions, we trained a learning algorithm (support vector machine, SVM). The SVM (as discussed by F. Model,P. Adorjan,A. Olek,C. Piepenbrock, Feature selection for DNA methylation based cancer classification. Bioinformatics. 2001 Jun;17 Suppl 1:S157-64) constructs an optimal discriminant between two classes of given training samples. In this case each sample is described by the methylation patterns (CG/TG ratios) at the investigated CpG sites. The SVM was trained on a subset of samples of each class, which were presented with the diagnosis attached. Independent test samples, which were not shown to the SVM before were then presented to evaluate, if the diagnosis can be predicted correctly based on the predictor created in the training round. This procedure was repeated several times using different partitions of the samples, a method called cross-validation. Please note that all rounds are performed without using any knowledge obtained in the previous runs. The number of correct classifications was averaged over all runs, which gives a good estimate of our test accuracy (percent of correct classified samples over all rounds).

### Healthy colon tissue compared to non healthy colon tissue (colon adenoma and colon carcinoma) (Figure 1)

Figure 1 shows the differentiation of healthy tissue from non healthy tissue wherein the non healthy specimens are obtained from either colon adenoma or colon carcinoma tissue. The evaluation is carried out using informative CpG positions from 27 genes. Informative CpG positions are further described in Table 3.

### Healthy colon tissue compared to colon carcinoma tissue (Figure 2)

Figure 2 shows the differentiation of healthy tissue from carcinoma tissue using informative CpG positions from 15 genes. Informative CpG positions are further described in Table 4.

### Healthy colon tissue compared to colon adenoma tissue (Figure 3)

Figure 3 shows the differentiation of healthy tissue from adenoma tissue using informative CpG positions from 40 genes. Informative CpG positions are further described in Table 5.

### Colon carcinoma tissue compared to colon adenoma tissue (Figures 4)

Figure 4 shows the differentiation of carcinoma tissue from adenoma tissue using informative CpG positions from 2 genes. Informative CpG positions are further described in Table 6.

### Example 3 : Identification of the methylation status of a CpG site within the gene CD44.

A fragment of the bisulfite treated DNA of the gene CD44 (Seq ID NO: 20) was PCR amplified using primers GAAAGGAGAGGTTAAAGGTTG (Seq ID NO 429) and AACTCACTTAACTCCAATCCC (Seq ID NO 430). The resultant fragment (696 bp in length) contained an informative CpG at position 235. The amplificate DNA was digested with the restriction endonuclease *Apa I,* recognition site GGGCC. Hydrolysis by said endonuclease is blocked by methylation of the CpG at position 235 of the amplificate. The digest was used as a control.

Genomic DNA was isolated from sample using the DNA wizzard DNA isolation kit (Promega). Each sample was digested using *Apa I* according to manufacturer's recommendations (New England Biolabs).

10 ng of each genomic digest was then amplified using PCR primers GAAAGGAGAGGTTAAAGGTTG and AACTCACTTAACTCCAATCCC. The PCR reactions were performed using a thermocycler (Eppendorf GmbH) using 10 ng of DNA, 6 pmol of each primer, 200 µM of each dNTP, 1.5 mM MgC12 and 1 U of HotstartTaq (Qiagen AG). The other conditions were as recommended by the Taq polymerase manufacturer. Using the above mentioned primers, gene fragments were amplified by PCR performing a first denaturation step for 14 min at 96 °C, followed by 30 - 45 cycles (step 2: 60 sec at 96°C, step 3: 45 sec at 52°C, step 4: 75 sec at 72°C) and a subsequent final elongation of 10 min at 72°C. The presence of PCR products was analysed by agarose gel electrophoresis.

PCR products were detectable with *Apa I* hydrolysed DNA isolated wherein the CpG position in question was up-methylated, when step 2 to step 4 of the cycle program were repeated 34, 37, 39, 42 and 45 fold. In contrast PCR products were only detectable with *Apa I* hydrolysed DNA isolated from down-methylated DNA (and control DNA) when step 2 to step 4 of the cycle program were repeated 42 and 45 fold. These results were incorporated into a CpG methylation matrix analysis as described in Example 2.

### Tables

**Table 1: PCR primers and products**

| ***No:*** | ***Gene:*** | ***Primer:*** | ***Amplificate Length:*** |
|---|---|---|---|
| 1 | MDR1 (SEQ ID NO: 1) | TAAGTATGTTGAAGAAAGATTATTGTAG (SEQ ID NO: 389) | 633 |
| | | TAAAAACTATCCCATAATAACTCCCAAC (SEQ ID NO: 390) | |
| 2 | APOC2 (SEQ ID NO: 2) | ATGAGTAGAAGAGGTGATAT (SEQ ID NO: 391) | 533 |
| | | CCCTAAATCCCTTTCTTACC (SEQ ID NO: 392) | |
| 3 | CACNA1G (SEQ ID NO: 3) | GGGATTTAAGAGAAATTGAGGTA (SEQ ID NO: 393) | 707 |
| | | AAACCCCAAACATCCTTTAT (SEQ ID NO: 394) | |
| 4 | EGR4 (SEQ ID NO: 4) | AGGGGGATTGAGTGTTAAGT (SEQ ID NO: 395) | 293 |
| | | CCCAAACATAAACACAAAAT (SEQ ID NO: 396) | |
| 5 | AR (SEQ ID NO: 5) | GTAGTAGTAGTAGTAAGAGA (SEQ ID NO: 397) | 460 |
| | | ACCCCCTAAATAATTATCCT (SEQ ID NO: 398) | |
| 6 | RB1 (SEQ ID NO: 6) | TTTAAGTTTGTTTTTGTTTTGGT (SEQ ID NO: 399) | 718 |
| | | TCCTACTCTAAATCCTCCTCAA (SEQ ID NO: 400) | |
| 7 | GPIb beta (SEQ ID NO: 7) | GGTGATAGGAGAATAATGTTGG (SEQ ID NO: 401) | 379 |
| | | TCTCCCAACTACAACCAAAC (SEQ ID NO: 402) | |
| 8 | MYOD1 (SEQ ID NO: 8) | ATTAGGGGTATAGAGGAGTATTGA (SEQ ID NO: 403) | 883 |
| | | CTTACAAACCCACAATAAACAA (SEQ ID NO: 404) | |
| 9 | WT1 (SEQ ID NO: 9) | AAAGGGAAATTAAGTGTTGT (SEQ ID NO: 405) | 747 |
| | | TAACTACCCTCAACTTCCC (SEQ ID NO: 406) | |
| 10 | HLA-F (SEQ ID NO: 10) | TTGTTGTTTTTAGGGGTTTTGG (SEQ ID NO: 407) | 946 |
| | | TCCTTCCCATTCTCCAAATATC (SEQ ID NO: 408) | |
| 11 | ELK1 (SEQ ID NO: 11) | AAGTGTTTTAGTTTTTAATGGGTA (SEQ ID NO: 409) | 966 |
| | | CAAACCCAAAACTCACCTAT (SEQ ID NO: 410) | |
| 12 | APC (SEQ ID NO: 12) | TCAACTACCATCAACTTCCTTA (SEQ ID NO: 411) | 491 |
| | | AATTTATTTTTAGTGTTGTAGTGGG (SEQ ID NO: 412) | |
| 13 | BCL2 (SEQ ID NO: 13) | GTATTTTATGTTAAGGGGGAAA (SEQ ID NO: 413) | 640 |
| | | AAAAACCACAATCCTCCC (SEQ ID NO: 414) | |
| 14 | CALCA (SEQ ID NO: 14) | GTTTTGGAAGTATGAGGGTG (SEQ ID NO: 415) | 614 |
| | | CCAAATTCTAAACCAATTTCC (SEQ ID NO: 416) | |
| 15 | CDH1 (SEQ ID NO: 15) | GAGGTTGGGGTTAGAGGAT (SEQ ID NO: 417) | 478 |
| | | CAAACTCACAAATACTTTACAATTC (SEQ ID NO: 418) | |
| 16 | CDKN1A (SEQ ID NO: 16) | GGATTAGTGGGAATAGAGGTG (SEQ ID NO: 419) | 408 |
| | | AAACCCAAACTCCTAACTACC (SEQ ID NO: 420) | |
| 17 | CDKN1B (p27 Kip1) (SEQ ID NO: 17) | GTGGGGAGGTAGTTGAAGA (SEQ ID NO: 421) | 478 |
| | | ATACACCCCTAACCCAAAAT (SEQ ID NO: 422) | |
| 18 | CDKN2a (SEQ ID NO: 18) | TTGAAAATTAAGGGTTGAGG (SEQ ID NO: 423) | 598 |
| | | CACCCTCTAATAACCAACCA (SEQ ID NO: 424) | |
| 19 | CDKN2a (SEQ ID NO: 18) | GGGGTTGGTTGGTTATTAGA (SEQ ID NO: 425) | 256 |
| | | AACCCTCTACCCACCTAAAT (SEQ ID NO: 426) | |
| 20 | CDKN2B (SEQ ID NO: 19) | GGTTGGTTGAAGGAATAGAAAT (SEQ ID NO: 427) | 708 |
| | | CCCACTAAACATACCCTTATTC (SEQ ID NO: 428) | |
| 21 | CD44 (SEQ ID NO: 20) | GAAAGGAGAGGTTAAAGGTTG (SEQ ID NO: 429) | 696 |
| | | AACTCACTTAACTCCAATCCC (SEQ ID NO: 430) | |
| 22 | CSPG2 (SEQ ID NO: 21) | GGATAGGAGTTGGGATTAAGAT (SEQ ID NO: 431) | 414 |
| | | AAATCTTTTTCAACACCAAAAT (SEQ ID NO: 432) | |
| 23 | DAPK1 (SEQ ID NO: 22) | AACCCTTTCTTCAAATTACAAA (SEQ ID NO: 433) | 348 |
| | | TGATTGGGTTTTAGGGAAATA (SEQ ID NO: 434) | |
| 24 | EGFR (SEQ ID NO: 23) | GGGTTTGGTTGTAATATGGATT (SEQ ID NO: 435) | 732 |
| | | CCCAACACTACCCCTCTAA (SEQ ID NO: 436) | |
| 25 | EYA4 (SEQ ID NO: 24) | GGAAGAGGTGATTAAATGGAT (SEQ ID NO: 437) | 226 |
| | | CCCAAAAATCAAACAACAA (SEQ ID NO: 438) | |
| 26 | GSTP1 (SEQ ID NO: 25) | ATTTGGGAAAGAGGGAAAG (SEQ ID NO: 439) | 300 |
| | | TAAAAACTCTAAACCCCATCC (SEQ ID NO: 440) | |
| 27 | GTBP/MSH6 (SEQ ID NO: 26) | CCCTACCCACCAATATACC (SEQ ID NO: 441) | 278 |
| | | AGATTTGGGGAAGAAGTTGTA (SEQ ID NO: 442) | |
| 28 | HIC-1 (SEQ ID NO: 27) | TGGGTTGGAGAAGAAGTTTA (SEQ ID NO: 443) | 280 |
| | | TCATATTTCCAAAAACACACC (SEQ ID NO: 444) | |
| 29 | HRAS (SEQ ID NO: 28) | CTTATTCCCATCTAAACCCTATT (SEQ ID NO: 445) | 331 |
| | | GTGGTTTTGTGAAGTTTTAGGT (SEQ ID NO: 446) | |
| 30 | IGF2 (SEQ ID NO: 29) | CCCTTCCCCTTAACTAAACT (SEQ ID NO: 447) | 364 |
| | | AATTTGGGTTAGGTTTGGA (SEQ ID NO: 448) | |
| 31 | LKB1 (SEQ ID NO: 30) | TAAAAGAAGGATTTTTGATTGG (SEQ ID NO: 449) | 528 |
| | | CATCTTATTTACCTCCCTCCC (SEQ ID NO: 450) | |
| 32 | MGMT (SEQ ID NO: 31) | AAGGTTTTAGGGAAGAGTGTTT (SEQ ID NO: 451) | 636 |
| | | ACCTTTTCCTATCACAAAAATAA (SEQ ID NO: 452) | |
| 33 | MLH1 (SEQ ID NO: 32) | TAAGGGGAGAGGAGGAGTTT (SEQ ID NO: 453) | 545 |
| | | ACCAATTCTCAATCATCTCTTT (SEQ ID NO: 454) | |
| 34 | MNCA9 (SEQ ID NO: 33) | GGGAAGTAGGTTAGGGTTAGTT (SEQ ID NO: 455) | |
| | | AAATCCTCCTCTCCAAATAAAT (SEQ ID NO: 456) | 616 |
| 35 | MSH3 (SEQ ID NO: 34) | TGTTTGGGATTGGGTAGG (SEQ ID NO: 457) | 211 |
| | | CATAACCTTTACCTATCTCCTCA (SEQ ID NO: 458) | |
| 36 | MYC (SEQ ID NO: 35) | AGAGGGAGTAAAAGAAAATGGT (SEQ ID NO: 459) | 712 |
| | | CCAAATAAACAAAATAACCTCC (SEQ ID NO: 460) | |
| 37 | N33 (SEQ ID NO: 36) | TTTTAGATTGAGGTTTTAGGGT (SEQ ID NO: 461) | 497 |
| | | ATCCATTCTACCTCCTTTTTCT (SEQ ID NO: 462) | |
| 38 | PAX6 (SEQ ID NO: 37) | GGAGGGGAGAGGGTTATG (SEQ ID NO: 463) | 374 |
| | | TACTATACACACCCCAAAACAA (SEQ ID NO: 464) | |
| 39 | PGR (SEQ ID NO: 38) | TTTTGGGAATGGGTTGTAT (SEQ ID NO: 465) | 369 |
| | | CTACCCTTAACCTCCATCCTA (SEQ ID NO: 466) | |
| 40 | PTEN (SEQ ID NO: 39) | TTTTAGGTAGTTATATTGGGTATGTT (SEQ ID NO: 467) | 346 |
| | | TCAACTCTCAAACTTCCATCA (SEQ ID NO: 468) | |
| 41 | RARB (SEQ ID NO: 40) | TTGTTGGGAGTTTTTAAGTTTT (SEQ ID NO: 469) | 353 |
| | | CAAATTCTCCTTCCAAATAAAT (SEQ ID NO: 470) | |
| 42 | SFN (SEQ ID NO: 41) | GAAGAGAGGAGAGGGAGGTA (SEQ ID NO: 471) | 489 |
| | | CTATCCAACAAACCCAACA (SEQ ID NO: 472) | |
| 43 | S100A2 (SEQ ID NO: 42) | GTTTTTAAGTTGGAGAAGAGGA (SEQ ID NO: 473) | 460 |
| | | ACCTATAAATCACAACCCACTC (SEQ ID NO: 474) | |
| 44 | TGFBR2 (SEQ ID NO: 43) | GTAATTTGAAGAAAGTTGAGGG (SEQ ID NO: 475) | 296 |
| | | CCAACAACTAAACAAAACCTCT (SEQ ID NO: 476) | |
| 45 | TIMP3 (SEQ ID NO: 44) | TGAGAAAATTGTTGTTTGAAGT (SEQ ID NO: 477) | 306 |
| | | CAAAATACCCTAAAAACCACTC (SEQ ID NO: 478) | |
| 46 | TP53 (SEQ ID NO: 45) | GGAGTTGTATTGTTGGGAGA (SEQ ID NO: 479) | 279 |
| | | TAAAACCCCAATTTTCACTAA (SEQ ID NO: 480) | |
| 47 | TP73 (SEQ ID NO: 46) | AGTAAATAGTGGGTGAGTTATGAA (SEQ ID NO: 481) | 607 |
| | | GAAAAACCTCTAAAAACTACTCTCC (SEQ ID NO: 482) | |
| 48 | VHL (SEQ ID NO: 47) | TGTAAAATGAATAAAGTTAATGAGTG (SEQ ID NO: 483) | 362 |
| | | TCCTAAATTCAAATAATCCTCCT (SEQ ID NO: 484) | |
| 49 | CDKN1C (SEQ ID NO: 48) | GGGGAGGTAGATATTTGGATAA (SEQ ID NO: 485) | 300 |
| | | AACTACACCATTTATATTCCCAC (SEQ ID NO: 486) | |
| 50 | CAV1 (SEQ ID NO: 49) | GTTAGTATGTTTGGGGGTAAAT (SEQ ID NO: 487) | 435 |
| | | ATAAATAACACCTTCCACCCTA (SEQ ID NO: 488) | |
| 51 | CDH13 (SEQ ID NO: 50) | TTTGTATTAGGTTGGAAGTGGT (SEQ ID NO: 489) | 286 |
| | | CCCAAATAAATCAACAACAACA (SEQ ID NO: 490) | |
| 52 | DRGI (SEQ ID NO: 51) | GGTTTTGGGTTTAGTGGTAAAT (SEQ ID NO: 491) | 416 |
| | | AACTTTCATAACTCACCCTTTC (SEQ ID NO: 492) | |
| 53 | PTGS2 (SEQ ID NO: 52) | GATTTTTGGAGAGGAAGTTAAG (SEQ ID NO: 493) | 381 |
| | | AAAACTAAAAACCAAACCCATA (SEQ ID NO: 494) | |
| 54 | THBS1 (SEQ ID NO: 53) | TGGGGTTAGTTTAGGATAGG (SEQ ID NO: 495) | 398 |
| | | CTTAAAAACACTAAAACTTCTCAAA (SEQ ID NO: 496) | |
| 55 | TPEF (=TMEFF2; =HPP1) (SEQ ID NO: 54) | TTGTTTGGGTTAATAAATGGA (SEQ ID NO: 497) | 295 |
| | | CTTCTCTCTTCTCCCCTCTC (SEQ ID NO: 498) | |
| 56 | DNMT1 (SEQ ID NO: 55) | TCCCCATCACACCTAAAA (SEQ ID NO: 499) | 210 |
| | | GGGAGGAGGGGATGTATT (SEQ ID NO: 500) | |
| 57 | CEA (SEQ ID NO: 56) | TATGGGAGGAGGTTAGTAAGTG (SEQ ID NO: 501) | 680 |
| | | CCCCAAATCCTACATATAAAAA (SEQ ID NO: 502) | |
| 58 | MB (SEQ ID NO: 57) | GTTTTTGGTAAAGGGGTAGAA (SEQ ID NO: 503) | 598 |
| | | CCTAAAATATCAACCTCCACCT (SEQ ID NO: 504) | |
| 59 | PCNA (SEQ ID NO: 58) | TTTTTAGGTTGTAAGGAGGTTTT (SEQ ID NO: 505) | 608 |
| | | TAAATACCTCCAACACCTTTCT (SEQ ID NO: 506) | |
| 60 | CDC2 (SEQ ID NO: 59) | ATTAGAAGTGAAAGTAATGGAATTT (SEQ ID NO: 507) | 418 |
| | | TCAATTTCCAAAAACCAAC (SEQ ID NO: 508) | |
| 61 | ESR1 (SEQ ID NO: 60) | AGGGGGAATTAAATAGAAAGAG (SEQ ID NO: 509) | 662 |
| | | CAATAAAACCATCCCAAATACT (SEQ ID NO: 510) | |
| 62 | CASP8 (SEQ ID NO: 61) | AGTGGATTTGGAGTTTAGATGT (SEQ ID NO: 511) | 431 |
| | | AACAAAATAAAAACTTCTCCCA (SEQ ID NO: 512) | |
| 63 | RASSF1 (SEQ ID NO: 62) | ACCTCTCTACAAATTACAAATTCA (SEQ ID NO: 513) | 347 |
| | | AGTTTGGGTTAGTTTGGGTT (SEQ ID NO: 514) | |
| 64 | MSH4 (SEQ ID NO: 63) | AGGATGTTGAGGTTTGAGATT (SEQ ID NO: 515) | 339 |
| | | CACTATAATAACCACCACCCA (SEQ ID NO: 516) | |
| 65 | MSH5 (SEQ ID NO: 64) | TATTAGGAATAAAGTTGGGGAG (SEQ ID NO: 517) | 395 |
| | | AACCCTTCAAACAAAAATAAAA (SEQ ID NO: 518) | |

**Table 2: Hybridisation oligonucleotides**

| ***No:*** | ***Gene*** | ***Oligo:*** |
|---|---|---|
| 1 | MDR1 (SEQ ID NO: 1) | TTGGTGGTCGTTTTAAGG (SEQ ID NO: 519) |
| 2 | MDR1 (SEQ ID NO: 1) | TTGGTGGTTGTTTTAAGG (SEQ ID NO: 520) |
| 3 | MDR1 (SEQ ID NO: 1) | TTGAAAGACGTGTTTATA (SEQ ID NO: 521) |
| 4 | MDR1 (SEQ ID NO: 1) | TTGAAAGATGTGTTTATA (SEQ ID NO: 522) |
| 5 | MDR1 (SEQ ID NO: 1) | AGGTGTAACGGAAGTTAG (SEQ ID NO: 523) |
| 6 | MDR1 (SEQ ID NO: 1) | AGGTGTAATGGAAGTTAG (SEQ ID NO: 524) |
| 7 | MDR1 (SEQ ID NO: 1) | TAGTTTTTCGAGGAATTA (SEQ ID NO: 525) |
| 8 | MDR1 (SEQ ID NO: 1) | TAGTTTTTTGAGGAATTA (SEQ ID NO: 526) |
| 9 | APOC2 (SEQ ID NO: 2) | GAGAGTTTCGTTTTTGTT (SEQ ID NO: 527) |
| 10 | APOC2 (SEQ ID NO: 2) | GAGAGTTTTGTTTTTGTT (SEQ ID NO: 528) |
| 11 | APOC2 (SEQ ID NO: 2) | TTGGGGGACGTTATTGTT (SEQ ID NO: 529) |
| 12 | APOC2 (SEQ ID NO: 2) | TTGGGGGATGTTATTGTT (SEQ ID NO: 530) |
| 13 | APOC2 (SEQ ID NO: 2) | TGTGTTCGTTCGGAGTTG (SEQ ID NO: 531) |
| 14 | APOC2 (SEQ ID NO: 2) | TGTGTTTGTTTGGAGTTG (SEQ ID NO: 532) |
| 15 | APOC2 (SEQ ID NO: 2) | TGGGTTTGCGGAGAATGG (SEQ ID NO: 533) |
| 16 | APOC2 (SEQ ID NO: 2) | TGGGTTTGTGGAGAATGG (SEQ ID NO: 534) |
| 17 | CACNA1G (SEQ ID NO: 3) | TTTAGGAGCGTTAATGTG (SEQ ID NO: 535) |
| 18 | CACNA1G (SEQ ID NO: 3) | TTTAGGAGTGTTAATGTG (SEQ ID NO: 536) |
| 19 | CACNA1G (SEQ ID NO: 3) | TAGGGTTACGAGGTTAGG (SEQ ID NO: 537) |
| 20 | CACNA1 G (SEQ ID NO: 3) | TAGGGTTATGAGGTTAGG (SEQ ID NO: 538) |
| 21 | CACNA1G (SEQ ID NO: 3) | GGAGGTTACGTTTAGATT (SEQ ID NO: 539) |
| 22 | CACNA1G (SEQ ID NO: 3) | GGAGGTTATGTTTAGATT (SEQ ID NO: 540) |
| 23 | CACNA1G (SEQ ID NO: 3) | TTAGGGGTCGTGGATAAA (SEQ ID NO: 541) |
| 24 | CACNA1G (SEQ ID NO: 3) | TTAGGGGTTGTGGATAAA (SEQ ID NO: 542) |
| 25 | EGR4 (SEQ ID NO: 4) | GGTGGGAAGCGTATTTAT (SEQ ID NO: 543) |
| 26 | EGR4 (SEQ ID NO: 4) | GGTGGGAAGTGTATTTAT (SEQ ID NO: 544) |
| 27 | EGR4 (SEQ ID NO: 4) | TTATAGTTCGAGTTTTTT (SEQ ID NO: 545) |
| 28 | EGR4 (SEQ ID NO: 4) | TTATAGTTTGAGTTTTTT (SEQ ID NO: 546) |
| 29 | EGR4 (SEQ ID NO: 4) | GGAGTTTTCGGTATATAT (SEQ ID NO: 927) |
| 30 | EGR4 (SEQ ID NO: 4) | GGAGTTTTTGGTATATAT (SEQ ID NO: 928) |
| 31 | AR (SEQ ID NO: 5) | TGTTATTTCGAGAGAGGT (SEQ ID NO: 547) |
| 32 | AR (SEQ ID NO: 5) | TGTTATTTTGAGAGAGGT (SEQ ID NO: 548) |
| 33 | AR (SEQ ID NO: 5) | AGAGGTTGCGTTTTAGAG (SEQ ID NO: 1027) |
| 34 | AR (SEQ ID NO: 5) | AGAGGTTGTGTTTTAGAG (SEQ ID NO: 1028) |
| 35 | AR (SEQ ID NO: 5) | ATTTTGAGCGAGGTTAGT (SEQ ID NO: 549) |
| 36 | AR (SEQ ID NO: 5) | ATTTTGAGTGAGGTTAGT (SEQ ID NO: 550) |
| 37 | AR (SEQ ID NO: 5) | GTAGTATTCGAAGGTAGT (SEQ ID NO: 551) |
| 38 | AR (SEQ ID NO: 5) | GTAGTATTTGAAGGTAGT (SEQ ID NO: 552) |
| 39 | RB1 (SEQ ID NO: 6) | TTAGATTTCGGGATAGGG (SEQ ID NO: 553) |
| 40 | RB1 (SEQ ID NO: 6) | TTAGATTTTGGGATAGGG (SEQ ID NO: 554) |
| 41 | RB1 (SEQ ID NO: 6) | TATAGTTTCGTTAAGTGT (SEQ ID NO: 555) |
| 42 | RB1 (SEQ ID NO: 6) | TATAGTTTTGTTAAGTGT (SEQ ID NO: 556) |
| 43 | RB1 (SEQ ID NO: 6) | GTGTATTTCGGTTTGGAG (SEQ ID NO: 557) |
| 44 | RB1 (SEQ ID NO: 6) | GTGTATTTTGGTTTGGAG (SEQ ID NO: 558) |
| 45 | RB1 (SEQ ID NO: 6) | TGGATTTACGTTAGGTTT (SEQ ID NO: 559) |
| 46 | RB1 (SEQ ID NO: 6) | TGGATTTATGTTAGGTTT (SEQ ID NO: 560) |
| 47 | GPIb (SEQ ID NO: 7) | betaTGTTATTTGTCGTTGTAG (SEQ ID NO: 561) |
| 48 | GPIb beta (SEQ ID NO: 7) | TGTTATTTGTTGTTGTAG (SEQ ID NO: 562) |
| 49 | GPIb beta (SEQ ID NO: 7) | GTGGGAGCGGAAGTTTGA (SEQ ID NO: 563) |
| 50 | GPIb (SEQ ID NO: 7) | betaGTGGGAGTGGAAGTTTGA (SEQ ID NO: 564) |
| 51 | GPIb beta (SEQ ID NO: 7) | TAGAGTAAGTCGGGTTGT (SEQ ID NO: 565) |
| 52 | GPIb beta (SEQ ID NO: 7) | TAGAGTAAGTCGGGTTGTT (SEQ ID NO: 566) |
| 53 | GPIb beta (SEQ ID NO: 7) | GGTTAGGTCGTAGTATTG (SEQ ID NO: 567) |
| 54 | GPIb beta (SEQ ID NO: 7) | GGTTAGGTTGTAGTATTG (SEQ ID NO: 568) |
| 55 | GPIb beta (SEQ ID NO: 7) | GGAGTTCGGTCGGGTTTT (SEQ ID NO: 1005) |
| 56 | GPIb (SEQ ID NO: 7) | betaGGAGTTTGGTTGGGTTTT (SEQ ID NO: 1006) |
| 57 | MYOD1 (SEQ ID NO: 8) | ATAGTAGTCGGGTGTTGG (SEQ ID NO: 569) |
| 58 | MYOD1 (SEQ ID NO: 8) | ATAGTAGTTGGGTGTTGG (SEQ ID NO: 570) |
| 59 | MYOD1 (SEQ ID NO: 8) | GTGTTAGTCGTTTAGGGT (SEQ ID NO: 1009) |
| 60 | MYOD1 (SEQ ID NO: 8) | GTGTTAGTTGTTTAGGGT (SEQ ID NO: 1010) |
| 61 | MYOD1 (SEQ ID NO: 8) | TAGTTGTTCGTTTGGGTT (SEQ ID NO: 571) |
| 62 | MYOD1 (SEQ ID NO: 8) | TAGTTGTTTGTTTGGGTT (SEQ ID NO: 572) |
| 63 | MYOD1 (SEQ ID NO: 8) | AATTAGGTCGGATAGGAG (SEQ ID NO: 975) |
| 64 | MYOD1 (SEQ ID NO: 8) | AATTAGGTTGGATAGGAG (SEQ ID NO: 976) |
| 65 | WT1 (SEQ ID NO: 9) | TAGTGAGACGAGGTTTTT (SEQ ID NO: 1017) |
| 66 | WT1 (SEQ ID NO: 9) | TAGTGAGATGAGGTTTTT (SEQ ID NO: 1018) |
| 67 | WT1 (SEQ ID NO: 9) | TATATTGGCGAAGGTTAA (SEQ ID NO: 967) |
| 68 | WT1 (SEQ ID NO: 9) | TATATTGGTGAAGGTTAA (SEQ ID NO: 968) |
| 69 | WT1 (SEQ ID NO: 9) | TGTTATATCGGTTAGTTG (SEQ ID NO: 959) |
| 70 | WT1 (SEQ ID NO: 9) | TGTTATATTGGTTAGTTG (SEQ ID NO: 960) |
| 71 | WT1 (SEQ ID NO: 9) | TTTAGTTTCGATTTTTGG (SEQ ID NO: 573) |
| 72 | WT1 (SEQ ID NO: 9) | TTTAGTTTTGATTTTTGG (SEQ ID NO: 574) |
| 73 | HLA-F (SEQ ID NO: 10) | ATAGGGTACGTTAAGGTT (SEQ ID NO: 575) |
| 74 | HLA-F (SEQ ID NO: 10) | ATAGGGTATGTTAAGGTT (SEQ ID NO: 576) |
| 75 | HLA-F (SEQ ID NO: 10) | TATTTGGGCGGGTGAGTG (SEQ ID NO: 939) |
| 76 | HLA-F (SEQ ID NO: 10) | TATTTGGGTGGGTGAGTG (SEQ ID NO: 940) |
| 77 | HLA-F (SEQ ID NO: 10) | GAGAGAAACGGTTTTTGT (SEQ ID NO: 577) |
| 78 | HLA-F (SEQ ID NO: 10) | GAGAGAAATGGTTTTTGT (SEQ ID NO: 578) |
| 79 | HLA-F (SEQ ID NO: 10) | AGTTGTTTCGTAGATATT (SEQ ID NO: 989) |
| 80 | HLA-F (SEQ ID NO: 10) | AGTTGTTTTGTAGATATT (SEQ ID NO: 990) |
| 81 | ELK1 (SEQ ID NO: 11) | TGTTTAATCGTAGAGTTG (SEQ ID NO: 579) |
| 82 | ELK1 (SEQ ID NO: 11) | TGTTTAATTGTAGAGTTG (SEQ ID NO: 580) |
| 83 | ELK1 (SEQ ID NO: 11) | TTTGTTTTCGTTGAGTAG (SEQ ID NO: 581) |
| 84 | ELK1 (SEQ ID NO: 11) | TTTGTTTTTGTTGAGTAG (SEQ ID NO: 582) |
| 85 | ELK1 (SEQ ID NO: 11) | GAAGGGTTCGTTTTTTAA (SEQ ID NO: 583) |
| 86 | ELK1 (SEQ ID NO: 11) | GAAGGGTTTGTTTTTTAA (SEQ ID NO: 584) |
| 87 | ELK1 (SEQ ID NO: 11) | ATTAATAGCGTTTTGGTT (SEQ ID NO: 585) |
| 88 | ELK1 (SEQ ID NO: 11) | ATTAATAGTGTTTTGGTT (SEQ ID NO: 586) |
| 89 | APC (SEQ ID NO: 12) | TTTAATCGTATAGTTTGT (SEQ ID NO: 971) |
| 90 | APC (SEQ ID NO: 12) | TTTAATTGTATAGTTTGT (SEQ ID NO: 972) |
| 91 | APC (SEQ ID NO: 12) | TATTTAGCGGATTATATA (SEQ ID NO: 587) |
| 92 | APC (SEQ ID NO: 12) | TATTTAGTGGATTATATA (SEQ ID NO: 588) |
| 93 | APC (SEQ ID NO: 12) | TATTTTGGCGGGTTGTAT (SEQ ID NO: 985) |
| 94 | APC (SEQ ID NO: 12) | TATTTTGGTGGGTTGTAT (SEQ ID NO: 986) |
| 95 | APC (SEQ ID NO: 12) | AAGGTTATCGGTTTAAGA (SEQ ID NO: 589) |
| 96 | APC (SEQ ID NO: 12) | AAGGTTATTGGTTTAAGA (SEQ ID NO: 590) |
| 97 | APC (SEQ ID NO: 12) | GGGGGACGACGTTTTTGT (SEQ ID NO: 591) |
| 98 | APC (SEQ ID NO: 12) | GGGGGATGATGTTTTTGT (SEQ ID NO: 592) |
| 99 | BCL2 (SEQ ID NO: 13) | AGTGTTTCGCGTGATTGA (SEQ ID NO: 593) |
| 100 | BCL2 (SEQ ID NO: 13) | AGTGTTTTGTGTGATTGA (SEQ ID NO: 594) |
| 101 | BCL2 (SEQ ID NO: 13) | TAAGTTGTCGTAGAGGGG (SEQ ID NO: 595) |
| 102 | BCL2 (SEQ ID NO: 13) | TAAGTTGTTGTAGAGGGG (SEQ ID NO: 596) |
| 103 | BCL2 (SEQ ID NO: 13) | GGATTTCGTCGTTGTAGA (SEQ ID NO: 597) |
| 104 | BCL2 (SEQ ID NO: 13) | GGATTTTGTTGTTGTAGA (SEQ ID NO: 598) |
| 105 | BCL2 (SEQ ID NO: 13) | TTTTGTTACGGTGGTGGA (SEQ ID NO: 1025) |
| 106 | BCL2 (SEQ ID NO: 13) | TTTTGTTATGGTGGTGGA (SEQ ID NO: 1026) |
| 107 | CALCA (SEQ ID NO: 14) | GAGGGTGACGTAATTTAG (SEQ ID NO: 599) |
| 108 | CALCA (SEQ ID NO: 14) | GAGGGTGATGTAATTTAG (SEQ ID NO: 600) |
| 109 | CALCA (SEQ ID NO: 14) | TGTATTGGCGGAATTTTT (SEQ ID NO: 601) |
| 110 | CALCA (SEQ ID NO: 14) | TGTATTGGTGGAATTTTT (SEQ ID NO: 602) |
| 111 | CALCA (SEQ ID NO: 14) | ATTAGGTTCGTGTTTTAG (SEQ ID NO: 953) |
| 112 | CALCA (SEQ ID NO: 14) | ATTAGGTTTGTGTTTTAG (SEQ ID NO: 954) |
| 113 | CALCA (SEQ ID NO: 14) | GTTAGTTTCGGGATATTT (SEQ ID NO: 603) |
| 114 | CALCA (SEQ ID NO: 14) | GTTAGTTTTGGGATATTT (SEQ ID NO: 604) |
| 115 | CDH1 (SEQ ID NO: 15) | TAGAGGATCGTTTGAGTT (SEQ ID NO: 605) |
| 116 | CDH1 (SEQ ID NO: 15) | TAGAGGATTGTTTGAGTT (SEQ ID NO: 606) |
| 117 | CDH1 (SEQ ID NO: 15) | GTTGTGATCGTATTATTG (SEQ ID NO: 607) |
| 118 | CDH1 (SEQ ID NO: 15) | GTTGTGATTGTATTATTG (SEQ ID NO: 608) |
| 119 | CDH1 (SEQ ID NO: 15) | TTGGGATTCGAATTTAGT (SEQ ID NO: 609) |
| 120 | CDH1 (SEQ ID NO: 15) | TTGGGATTTGAATTTAGT (SEQ ID NO: 610) |
| 121 | CDH1 (SEQ ID NO: 15) | AGGGTTATCGCGTTTATG (SEQ ID NO: 983) |
| 122 | CDH1 (SEQ ID NO: 15) | AGGGTTATTGTGTTTATG (SEQ ID NO: 984) |
| 123 | CDH1 (SEQ ID NO: 15) | TAGTGGCGTCGGAATTGT (SEQ ID NO: 929) |
| 124 | CDH1 (SEQ ID NO: 15) | TAGTGGTGTTGGAATTGT (SEQ ID NO: 930) |
| 125 | CDKN1A (SEQ ID NO: 16) | AGGTGTATCGTTTTTATA (SEQ ID NO: 611) |
| 126 | CDKN1A (SEQ ID NO: 16) | AGGTGTATTGTTTTTATA (SEQ ID NO: 612) |
| 127 | CDKN1A (SEQ ID NO: 16) | TGGGTTAGCGGTGAGTTA (SEQ ID NO: 613) |
| 128 | CDKN1A (SEQ ID NO: 16) | TGGGTTAGTGGTGAGTTA (SEQ ID NO: 614) |
| 129 | CDKN1A (SEQ ID NO: 16) | GTTTATTTCGTGGGGAAA (SEQ ID NO: 615) |
| 130 | CDKN1A (SEQ ID NO: 16) | GTTTATTTTGTGGGGAAA (SEQ ID NO: 616) |
| 131 | CDKN1A (SEQ ID NO: 16) | TTGGAATTCGGTTAGGTT (SEQ ID NO: 617) |
| 132 | CDKN1A (SEQ ID NO: 16) | TTGGAATTTGGTTAGGTT (SEQ ID NO: 618) |
| 133 | CDKN1B (p27 Kip1) (SEQ ID NO: 17) | AAGAGAAACGTTGGAATA (SEQ ID NO: 619) |
| 134 | CDKN1B (p27Kip1) (SEQ ID NO: 17) | AAGAGAAATGTTGGAATA (SEQ ID NO: 620) |
| 135 | CDKN1B (p27Kip1) (SEQ ID NO: 17) | TTTGATTTCGAGGGGAGT (SEQ ID NO: 621) |
| 136 | CDKN1B (p2Kip1) (SEQ ID NO: 17) | 7TTTGATTTTGAGGGGAGT (SEQ ID NO: 622) |
| 137 | CDKN1B (p27 Kip1) (SEQ ID NO: 17) | GTATTTGGCGGTTGGATT (SEQ ID NO: 623) |
| 138 | CDKN1B (p27 Kip1) (SEQ ID NO: 17) | GTATTTGGTGGTTGGATT (SEQ ID NO: 624) |
| 139 | CDKN1B (p27Kip1) (SEQ ID NO: 17) | TATAATTTCGGGAAAGAA (SEQ ID NO: 625) |
| 140 | CDKN1B (p27 Kip1) (SEQ ID NO: 17) | TATAATTTTGGGAAAGAA (SEQ ID NO: 626) |
| 141 | CDKN2a (SEQ ID NO: 18) | AGAGTGAACGTATTTAAA (SEQ ID NO: 627) |
| 142 | CDKN2a (SEQ ID NO: 18) | AGAGTGAATGTATTTAAA (SEQ ID NO: 628) |
| 143 | CDKN2a (SEQ ID NO: 18) | GTTGTTTTCGGTTGGTGT (SEQ ID NO: 1029) |
| 144 | CDKN2a (SEQ ID NO: 18) | GTTGTTTTTGGTTGGTGT (SEQ ID NO: 1030) |
| 145 | CDKN2a (SEQ ID NO: 18) | GATAGGGTCGGAGGGGGT (SEQ ID NO: 629) |
| 146 | CDKN2a (SEQ ID NO: 18) | GATAGGGTTGGAGGGGGT (SEQ ID NO: 630) |
| 147 | CDKN2a (SEQ ID NO: 18) | GGAGTTTTCGGTTGATTG (SEQ ID NO: 997) |
| 148 | CDKN2a (SEQ ID NO: 18) | GGAGTTTTTGGTTGATTG (SEQ ID NO: 998) |
| 149 | CDKN2a (SEQ ID NO: 18) | AATAGTTACGGTCGGAGG (SEQ ID NO: 981) |
| 150 | CDKN2a (SEQ ID NO: 18) | AATAGTTATGGTTGGAGG (SEQ ID NO: 982) |
| 151 | CDKN2B (SEQ ID NO: 19) | ATATTTAGCGAGTAGTGT (SEQ ID NO: 631) |
| 152 | CDKN2B (SEQ ID NO: 19) | ATATTTAGTGAGTAGTGT (SEQ ID NO: 632) |
| 153 | CDKN2B (SEQ ID NO: 19) | ATAGGGGGCGGAGTTTAA (SEQ ID NO: 633) |
| 154 | CDKN2B (SEQ ID NO: 19) | ATAGGGGGTGGAGTTTAA (SEQ ID NO: 634) |
| 155 | CDKN2B (SEQ ID NO: 19) | TTATTGTACGGGGTTTTA (SEQ ID NO: 635) |
| 156 | CDKN2B (SEQ ID NO: 19) | TTATTGTATGGGGTTTTA (SEQ ID NO: 636) |
| 157 | CDKN2B (SEQ ID NO: 19) | TTTTAAGTCGTAGAAGGA (SEQ ID NO: 637) |
| 158 | CDKN2B (SEQ ID NO: 19) | TTTTAAGTTGTAGAAGGA (SEQ ID NO: 638) |
| 159 | CD44 (SEQ ID NO: 20) | GTGGGGTTCGGAGGTATA (SEQ ID NO: 919) |
| 160 | CD44 (SEQ ID NO: 20) | GTGGGGTTTGGAGGTATA (SEQ ID NO: 920) |
| 161 | CD44 (SEQ ID NO: 20) | GGTAGTTTCGATTATTTA (SEQ ID NO: 639) |
| 162 | CD44 (SEQ ID NO: 20) | GGTAGTTTTGATTATTTA (SEQ ID NO: 640) |
| 163 | CD44 (SEQ ID NO: 20) | TTGTTTAGCGGATTTTAG (SEQ ID NO: 897) |
| 164 | CD44 (SEQ ID NO: 20) | TTGTTTAGTGGATTTTAG (SEQ ID NO: 898) |
| 165 | CD44 (SEQ ID NO: 20) | TGGTGGTACGTAGTTTGG (SEQ ID NO: 641) |
| 166 | CD44 (SEQ ID NO: 20) | TGGTGGTATGTAGTTTGG (SEQ ID NO: 642) |
| 167 | CSPG2 (SEQ ID NO: 21) | AAGATTTTCGGTTAGTTT (SEQ ID NO: 963) |
| 168 | CSPG2 (SEQ ID NO: 21) | AAGATTTTTGGTTAGTTT (SEQ ID NO: 964) |
| 169 | CSPG2 (SEQ ID NO: 21) | ATGTGATTCGTTTGGGTA (SEQ ID NO: 643) |
| 170 | CSPG2 (SEQ ID NO: 21) | ATGTGATTTGTTTGGGTA (SEQ ID NO: 644) |
| 171 | CSPG2 (SEQ ID NO: 21) | GGGTAACGTCGAATTTAG (SEQ ID NO: 901) |
| 172 | CSPG2 (SEQ ID NO: 21) | GGGTAATGTTGAATTTAG (SEQ ID NO: 902) |
| 173 | CSPG2 (SEQ ID NO: 21) | AAAAATTCGCGAGTTTAG (SEQ ID NO: 945) |
| 174 | CSPG2 (SEQ ID NO: 21) | AAAAATTTGTGAGTTTAG (SEQ ID NO: 946) |
| 175 | DAPK1 (SEQ ID NO: 22) | GTTGGAGTCGAGGTTTGA (SEQ ID NO: 645) |
| 176 | DAPK1 (SEQ ID NO: 22) | GTTGGAGTTGAGGTTTGA (SEQ ID NO: 646) |
| 177 | DAPK1 (SEQ ID NO: 22) | TTTTTTGTCGGATTGGTG (SEQ ID NO: 647) |
| 178 | DAPK1 (SEQ ID NO: 22) | TTTTTTGTTGGATTGGTG (SEQ ID NO: 648) |
| 179 | DAPK1 (SEQ ID NO: 22) | GAAGGGAGCGTATTTTAT (SEQ ID NO: 955) |
| 180 | DAPK1 (SEQ ID NO: 22) | GAAGGGAGTGTATTTTAT (SEQ ID NO: 956) |
| 181 | DAPK1 (SEQ ID NO: 22) | TTGTTTTTCGGAAATTTG (SEQ ID NO: 935) |
| 182 | DAPK1 (SEQ ID NO: 22) | TTGTTTTTTGGAAATTTG (SEQ ID NO: 936) |
| 183 | EGFR (SEQ ID NO: 23) | TTTGTATTCGGAGTTGGG (SEQ ID NO: 961) |
| 184 | EGFR (SEQ ID NO: 23) | TTTGTATTTGGAGTTGGG (SEQ ID NO: 962) |
| 185 | EGFR (SEQ ID NO: 23) | GATGATTTCGAGGGTGTT (SEQ ID NO: 649) |
| 186 | EGFR (SEQ ID NO: 23) | GATGATTTTGAGGGTGTT (SEQ ID NO: 650) |
| 187 | EGFR (SEQ ID NO: 23) | GAGGGTTTCGTAGTGTTG (SEQ ID NO: 651) |
| 188 | EGFR (SEQ ID NO: 23) | GAGGGTTTTGTAGTGTTG (SEQ ID NO: 652) |
| 189 | EGFR (SEQ ID NO: 23) | TGGGGATTCGAATAAAGG (SEQ ID NO: 653) |
| 190 | EGFR (SEQ ID NO: 23) | TGGGGATTTGAATAAAGG (SEQ ID NO: 654) |
| 191 | EGFR (SEQ ID NO: 23) | ATTTGGTTCGATTTGGAT (SEQ ID NO: 931) |
| 192 | EGFR (SEQ ID NO: 23) | ATTTGGTTTGATTTGGAT (SEQ ID NO: 932) |
| 193 | EGFR (SEQ ID NO: 23) | TATATATACGTGTGGGTA (SEQ ID NO: 655) |
| 194 | EYA4 (SEQ ID NO: 24) | TATATATATGTGTGGGTA (SEQ ID NO: 656) |
| 195 | EYA4 (SEQ ID NO: 24) | AGTGTATGCGTAGAAGGT (SEQ ID NO: 923) |
| 196 | EYA4 (SEQ ID NO: 24) | AGTGTATGTGTAGAAGGT (SEQ ID NO: 924) |
| 197 | EYA4 (SEQ ID NO: 24) | TTTAGATACGAAATGTTA (SEQ ID NO: 657) |
| 198 | EYA4 (SEQ ID NO: 24) | TTTAGATATGAAATGTTA (SEQ ID NO: 658) |
| 199 | EYA4 (SEQ ID NO: 24) | AAGTAAGTCGTTGTTGTT (SEQ ID NO: 921) |
| 200 | EYA4 (SEQ ID NO: 24) | AAGTAAGTTGTTGTTGTT (SEQ ID NO: 922) |
| 201 | GSTP1 (SEQ ID NO: 25) | GGTTTTTTCGGTTAGTTG (SEQ ID NO: 659) |
| 202 | GSTP1 (SEQ ID NO: 25) | GGTTTTTTTGGTTAGTTG (SEQ ID NO: 660) |
| 203 | GSTP1 (SEQ ID NO: 25) | GGAGTTCGCGGGATTTTT (SEQ ID NO: 905) |
| 204 | GSTP1 (SEQ ID NO: 25) | GGAGTTTGTGGGATTTTT (SEQ ID NO: 906) |
| 205 | GSTP1 (SEQ ID NO: 25) | GTAGTTTTCGTTATTAGT (SEQ ID NO: 661) |
| 206 | GSTP1 (SEQ ID NO: 25) | GTAGTTTTTGTTATTAGT (SEQ ID NO: 662) |
| 207 | GTBP/MSH6 (SEQ ID NO: 26) | GAGGAATTCGGGTTTTAG (SEQ ID NO: 951) |
| 208 | GTBP/MSH6 (SEQ ID NO: 26) | GAGGAATTTGGGTTTTAG (SEQ ID NO: 952) |
| 209 | GTBP/MSH6 (SEQ ID NO: 26) | TTTGTTGGCGGGAAATTT (SEQ ID NO: 925) |
| 210 | GTBP/MSH6 (SEQ ID NO: 26) | TTTGTTGGTGGGAAATTT (SEQ ID NO: 926) |
| 211 | GTBP/MSH6 (SEQ ID NO: 26) | TTTTGTCGGACGGAGTTT (SEQ ID NO: 663) |
| 212 | GTBP/MSH6 (SEQ ID NO: 26) | TTTTGTTGGATGGAGTTT (SEQ ID NO: 664) |
| 213 | GTBP/MSH6 (SEQ ID NO: 26) | AAGGTTTAATCGTTTTGT (SEQ ID NO: 665) |
| 214 | GTBP/MSH6 (SEQ ID NO: 26) | AAGGTTTAATTGTTTTGT (SEQ ID NO: 666) |
| 215 | HIC-1 (SEQ ID NO: 27) | TTAAAACGGCGTATAGGG (SEQ ID NO: 667) |
| 216 | HIC-1 (SEQ ID NO: 27) | TTAAAATGGTGTATAGGG (SEQ ID NO: 668) |
| 217 | HIC-1 (SEQ ID NO: 27) | AGGAGATTCGAAAGTTTA (SEQ ID NO: 669) |
| 218 | HIC-1 (SEQ ID NO: 27) | AGGAGATTTGAAAGTTTA (SEQ ID NO: 670) |
| 219 | HIC-1 (SEQ ID NO: 27) | TTTTAGAGCGTTAGGGTT (SEQ ID NO: 1021) |
| 220 | HIC-1 (SEQ ID NO: 27) | TTTTAGAGTGTTAGGGTT (SEQ ID NO: 1022) |
| 221 | HRAS (SEQ ID NO: 28) | ATAGTGGGCGTAATTGGT (SEQ ID NO: 671) |
| 222 | HRAS (SEQ ID NO: 28) | ATAGTGGGTGTAATTGGT (SEQ ID NO: 672) |
| 223 | HRAS (SEQ ID NO: 28) | AAATTGGACGTTTAGTTG (SEQ ID NO: 673) |
| 224 | HRAS (SEQ ID NO: 28) | AAATTGGATGTTTAGTTG (SEQ ID NO: 674) |
| 225 | HRAS (SEQ ID NO: 28) | TAGAAGTCGAGAGATTTG (SEQ ID NO: 675) |
| 226 | HRAS (SEQ ID NO: 28) | TAGAAGTTGAGAGATTTG (SEQ ID NO: 676) |
| 227 | HRAS (SEQ ID NO: 28) | GAATATTTCGAAGTTTGT (SEQ ID NO: 677) |
| 228 | HRAS (SEQ ID NO: 28) | GAATATTTTGAAGTTTGT (SEQ ID NO: 678) |
| 229 | IGF2 (SEQ ID NO: 29) | AGTTTGAACGATGTAAGA (SEQ ID NO: 973) |
| 230 | IGF2 (SEQ ID NO: 29) | AGTTTGAATGATGTAAGA (SEQ ID NO: 974) |
| 231 | IGF2 (SEQ ID NO: 29) | GGTTATTACGATAATTTG (SEQ ID NO: 679) |
| 232 | IGF2 (SEQ ID NO: 29) | GGTTATTATGATAATTTG (SEQ ID NO: 680) |
| 233 | IGF2 (SEQ ID NO: 29) | TTGTATGGTCGAGTTTAT (SEQ ID NO: 941) |
| 234 | IGF2 (SEQ ID NO: 29) | TTGTATGGTTGAGTTTAT (SEQ ID NO: 942) |
| 235 | IGF2 (SEQ ID NO: 29) | GATTAGGGCGGGAAATAT (SEQ ID NO: 937) |
| 236 | IGF2 (SEQ ID NO: 29) | GATTAGGGTGGGAAATAT (SEQ ID NO: 938) |
| 237 | IGF2 (SEQ ID NO: 29) | TGGAGTTTACGGAGGTTT (SEQ ID NO: 681) |
| 238 | IGF2 (SEQ ID NO: 29) | TGGAGTTTATGGAGGTTT (SEQ ID NO: 682) |
| 239 | LKB1 (SEQ ID NO: 30) | TTAATTAACGGGTGGGTA (SEQ ID NO: 683) |
| 240 | LKB1 (SEQ ID NO: 30) | TTAATTAATGGGTGGGTA (SEQ ID NO: 684) |
| 241 | LKB1 (SEQ ID NO: 30) | TTTAGGTTCGTAAGTTTA (SEQ ID NO: 965) |
| 242 | LKB1 (SEQ ID NO: 30) | TTTAGGTTTGTAAGTTTA (SEQ ID NO: 966) |
| 243 | LKB1 (SEQ ID NO: 30) | AGGGAGGTCGTTGGTATT (SEQ ID NO: 933) |
| 244 | LKB1 (SEQ ID NO: 30) | AGGGAGGTTGTTGGTATT (SEQ ID NO: 934) |
| 245 | MGMT (SEQ ID NO: 31) | TAAGGATACGAGTTATAT (SEQ ID NO: 685) |
| 246 | MGMT (SEQ ID NO: 31) | TAAGGATATGAGTTATAT (SEQ ID NO: 686) |
| 247 | MGMT (SEQ ID NO: 31) | TTGGAGAGCGGTTGAGTT (SEQ ID NO: 687) |
| 248 | MGMT (SEQ ID NO: 31) | TTGGAGAGTGGTTGAGTT (SEQ ID NO: 688) |
| 249 | MGMT (SEQ ID NO: 31) | TAGGTTATCGGTGATTGT (SEQ ID NO: 689) |
| 250 | MGMT (SEQ ID NO: 31) | TAGGTTATTGGTGATTGT (SEQ ID NO: 690) |
| 251 | MGMT (SEQ ID NO: 31) | TAGGGGAGCGGTTTTAGG (SEQ ID NO: 691) |
| 252 | MGMT (SEQ ID NO: 31) | TAGGGGAGTGGTTTTAGG (SEQ ID NO: 692) |
| 253 | MGMT (SEQ ID NO: 31) | AGTAGGATCGGGATTTTT (SEQ ID NO: 1001) |
| 254 | MGMT (SEQ ID NO: 31) | AGTAGGATTGGGATTTTT (SEQ ID NO: 1002) |
| 255 | MLH1 (SEQ ID NO: 32) | TTGAGAAGCGTTAAGTAT (SEQ ID NO: 693) |
| 256 | MLH1 (SEQ ID NO: 32) | TTGAGAAGTGTTAAGTAT (SEQ ID NO: 694) |
| 257 | MLH1 (SEQ ID NO: 32) | TTAGGTAGCGGGTAGTAG (SEQ ID NO: 949) |
| 258 | MLH1 (SEQ ID NO: 32) | TTAGGTAGTGGGTAGTAG (SEQ ID NO: 950) |
| 259 | MLH1 (SEQ ID NO: 32) | GTAGTAGTCGTTTTAGGG (SEQ ID NO: 695) |
| 260 | MLH1 (SEQ ID NO: 32) | GTAGTAGTTGTTTTAGGG (SEQ ID NO: 696) |
| 261 | MLH1 (SEQ ID NO: 32) | ATAGTTGTCGTTGAAGGG (SEQ ID NO: 697) |
| 262 | MLH1 (SEQ ID NO: 32) | ATAGTTGTTGTTGAAGGG (SEQ ID NO: 698) |
| 263 | MLH1 (SEQ ID NO: 32) | TTGGATGGCGTAAGTTAT (SEQ ID NO: 699) |
| 264 | MLH1 (SEQ ID NO: 32) | TTGGATGGTGTAAGTTAT (SEQ ID NO: 700) |
| 265 | MNCA9 (SEQ ID NO: 33) | TAAAAGGGCGTTTTGTGA (SEQ ID NO: 701) |
| 266 | MNCA9 (SEQ ID NO: 33) | TAAAAGGGTGTTTTGTGA (SEQ ID NO: 702) |
| 267 | MNCA9 (SEQ ID NO: 33) | TAGTTAGTCGTATGGTTT (SEQ ID NO: 703) |
| 268 | MNCA9 (SEQ ID NO: 33) | TAGTTAGTTGTATGGTTT (SEQ ID NO: 704) |
| 269 | MNCA9 (SEQ ID NO: 33) | GATTTATTCGGAGAGGAG (SEQ ID NO: 705) |
| 270 | MNCA9 (SEQ ID NO: 33) | GATTTATTTGGAGAGGAG (SEQ ID NO: 706) |
| 271 | MSH3 (SEQ ID NO: 34) | ATTTTTCGTTCGATGATA (SEQ ID NO: 707) |
| 272 | MSH3 (SEQ ID NO: 34) | ATTTTTTGTTTGATGATA (SEQ ID NO: 708) |
| 273 | MSH3 (SEQ ID NO: 34) | AGTTTAGTCGGGGTTATA (SEQ ID NO: 709) |
| 274 | MSH3 (SEQ ID NO: 34) | AGTTTAGTTGGGGTTATA (SEQ ID NO: 710) |
| 275 | MSH3 (SEQ ID NO: 34) | GGGTGAAGCGTTGAGGTT (SEQ ID NO: 711) |
| 276 | MSH3 (SEQ ID NO: 34) | GGGTGAAGTGTTGAGGTT (SEQ ID NO: 712) |
| 277 | MSH3 (SEQ ID NO: 34) | AGTATTTTCGTTTGAGGA (SEQ ID NO: 1015) |
| 278 | MSH3 (SEQ ID NO: 34) | AGTATTTTTGTTTGAGGA (SEQ ID NO: 1016) |
| 279 | MYC (SEQ ID NO: 35) | TTAGAGTGTTCGGTTGTT (SEQ ID NO: 713) |
| 280 | MYC (SEQ ID NO: 35) | TTAGAGTGTTTGGTTGTT (SEQ ID NO: 714) |
| 281 | MYC (SEQ ID NO: 35) | TTATAATGCGAGGGTTTG (SEQ ID NO: 1019) |
| 282 | MYC (SEQ ID NO: 35) | TTATAATGTGAGGGTTTG (SEQ ID NO: 1020) |
| 283 | MYC (SEQ ID NO: 35) | AGGATTTTCGAGTTGTGT (SEQ ID NO: 715) |
| 284 | MYC (SEQ ID NO: 35) | AGGATTTTTGAGTTGTGT (SEQ ID NO: 716) |
| 285 | MYC (SEQ ID NO: 35) | AATTTTAGCGAGAGGTAG (SEQ ID NO: 717) |
| 286 | MYC (SEQ ID NO: 35) | AATTTTAGTGAGAGGTAG (SEQ ID NO: 718) |
| 287 | N33 (SEQ ID NO: 36) | TTGGTTCGGGAAAGGTAA (SEQ ID NO: 977) |
| 288 | N33 (SEQ ID NO: 36) | TTGGTTTGGGAAAGGTAA (SEQ ID NO: 978) |
| 289 | N33 (SEQ ID NO: 36) | TGTTATTTCGGAGGGTTT (SEQ ID NO: 909) |
| 290 | N33 (SEQ ID NO: 36) | TGTTATTTTGGAGGGTTT (SEQ ID NO: 910) |
| 291 | N33 (SEQ ID NO: 36) | GTTTAGTTAGCGGGTTTT (SEQ ID NO: 943) |
| 292 | N33 (SEQ ID NO: 36) | GTTTAGTTAGTGGGTTTT (SEQ ID NO: 944) |
| 293 | N33 (SEQ ID NO: 36) | ATTTAGTTCGGGGGAGGA (SEQ ID NO: 993) |
| 294 | N33 (SEQ ID NO: 36) | ATTTAGTTTGGGGGAGGA (SEQ ID NO: 994) |
| 295 | PAX6 (SEQ ID NO: 37) | TATTGTTTCGGTTGTTAG (SEQ ID NO: 719) |
| 296 | PAX6 (SEQ ID NO: 37) | TATTGTTTTGGTTGTTAG (SEQ ID NO: 720) |
| 297 | PAX6 (SEQ ID NO: 37) | GTTAGTAGCGAGTTTAGG (SEQ ID NO: 721) |
| 298 | PAX6 (SEQ ID NO: 37) | GTTAGTAGTGAGTTTAGG (SEQ ID NO: 722) |
| 299 | PAX6 (SEQ ID NO: 37) | AGAGTTTAGCGTATTTTT (SEQ ID NO: 723) |
| 300 | PAX6 (SEQ ID NO: 37) | AGAGTTTAGTGTATTTTT (SEQ ID NO: 724) |
| 301 | PGR (SEQ ID NO: 38) | GAATTTAGCGAGGGATTG (SEQ ID NO: 725) |
| 302 | PGR (SEQ ID NO: 38) | GAATTTAGTGAGGGATTG (SEQ ID NO: 726) |
| 303 | PGR (SEQ ID NO: 38) | AGTATGTACGAGTTTGAT (SEQ ID NO: 727) |
| 304 | PGR (SEQ ID NO: 38) | AGTATGTATGAGTTTGAT (SEQ ID NO: 728) |
| 305 | PGR (SEQ ID NO: 38) | TTAAGTGTCGGATTTGTG (SEQ ID NO: 1011) |
| 306 | PGR (SEQ ID NO: 38) | TTAAGTGTTGGATTTGTG (SEQ ID NO: 1012) |
| 307 | PGR (SEQ ID NO: 38) | GGGATAAACGATAGTTAT (SEQ ID NO: 729) |
| 308 | PGR (SEQ ID NO: 38) | GGGATAAATGATAGTTAT (SEQ ID NO: 730) |
| 309 | PTEN (SEQ ID NO: 39) | AGAGTTTGCGGTTTGGGG (SEQ ID NO: 731) |
| 310 | PTEN (SEQ ID NO: 39) | AGAGTTTGTGGTTTGGGGT (SEQ ID NO: 732) |
| 311 | PTEN (SEQ ID NO: 39) | ATTTTGCGTTCGTATTTA (SEQ ID NO: 987) |
| 312 | PTEN (SEQ ID NO: 39) | ATTTTGTGTTTGTATTTA (SEQ ID NO: 988) |
| 313 | PTEN (SEQ ID NO: 39) | AGAGTTATCGTTTTGTTT (SEQ ID NO: 957) |
| 314 | PTEN (SEQ ID NO: 39) | AGAGTTATTGTTTTGTTT (SEQ ID NO: 958) |
| 315 | PTEN (SEQ ID NO: 39) | TGATGTGGCGGGATTTTT (SEQ ID NO: 947) |
| 316 | PTEN (SEQ ID NO: 39) | TGATGTGGTGGGATTTTT (SEQ ID NO: 948) |
| 317 | RARB (SEQ ID NO: 40) | TAGTAGTTCGGGTAGGGT (SEQ ID NO: 991) |
| 318 | RARB (SEQ ID NO: 40) | TAGTAGTTTGGGTAGGGT (SEQ ID NO: 992) |
| 319 | RARB (SEQ ID NO: 40) | GGGTTTATCGAAAGTTTA (SEQ ID NO: 733) |
| 320 | RARB (SEQ ID NO: 40) | GGGTTTATTGAAAGTTTA (SEQ ID NO: 734) |
| 321 | RARB (SEQ ID NO: 40) | AGTTTATTCGTATATATT (SEQ ID NO: 735) |
| 322 | RARB (SEQ ID NO: 40) | AGTTTATTTGTATATATT (SEQ ID NO: 736) |
| 323 | RARB (SEQ ID NO: 40) | TTTTTATGCGAGTTGTTT (SEQ ID NO: 737) |
| 324 | RARB (SEQ ID NO: 40) | TTTTTATGTGAGTTGTTT (SEQ ID NO: 738) |
| 325 | SFN (SEQ ID NO: 41) | ATAGAGTTCGGTATTGGT (SEQ ID NO: 739) |
| 326 | SFN (SEQ ID NO: 41) | ATAGAGTTTGGTATTGGT (SEQ ID NO: 740) |
| 327 | SFN (SEQ ID NO: 41) | GTAGGTCGAACGTTATGA (SEQ ID NO: 741) |
| 328 | SFN (SEQ ID NO: 41) | GTAGGTTGAATGTTATGA (SEQ ID NO: 742) |
| 329 | SFN (SEQ ID NO: 41) | AAAAGTAACGAGGAGGGT (SEQ ID NO: 743) |
| 330 | S100A2 (SEQ ID NO: 42) | AAAAGTAATGAGGAGGGT (SEQ ID NO: 744) |
| 331 | S100A2 (SEQ ID NO: 42) | TTTAATTGCGGTTGTGTG (SEQ ID NO: 745) |
| 332 | S100A2 (SEQ ID NO: 42) | TTTAATTGTGGTTGTGTG (SEQ ID NO: 746) |
| 333 | S100A2 (SEQ ID NO: 42) | TATATAGGCGTATGTATG (SEQ ID NO: 747) |
| 334 | S100A2 (SEQ ID NO: 42) | TATATAGGTGTATGTATG (SEQ ID NO: 748) |
| 335 | S100A2 (SEQ ID NO: 42) | TATGTATACGAGTATTGG (SEQ ID NO: 999) |
| 336 | S100A2 (SEQ ID NO: 42) | TATGTATATGAGTATTGG (SEQ ID NO: 1000) |
| 337 | S100A2 (SEQ ID NO: 42) | AGTTTTAGCGTGTGTTTA (SEQ ID NO: 749) |
| 338 | S100A2 (SEQ ID NO: 42) | AGTTTTAGTGTGTGTTTA (SEQ ID NO: 750) |
| 339 | TGFBR2 (SEQ ID NO: 43) | ATTTGGAGCGAGGAATTT (SEQ ID NO: 751) |
| 340 | TGFBR2 (SEQ ID NO: 43) | ATTTGGAGTGAGGAATTT (SEQ ID NO: 752) |
| 341 | TGFBR2 (SEQ ID NO: 43) | TTGAAAGTCGGTTAAAGT (SEQ ID NO: 753) |
| 342 | TGFBR2 (SEQ ID NO: 43) | TTGAAAGTTGGTTAAAGT (SEQ ID NO: 754) |
| 343 | TGFBR2 (SEQ ID NO: 43) | AAAGTTTTCGGAGGGGTT (SEQ ID NO: 907) |
| 344 | TGFBR2 (SEQ ID NO: 43) | AAAGTTTTTGGAGGGGTT (SEQ ID NO: 908) |
| 345 | TGFBR2 (SEQ ID NO: 43) | GGTAGTTACGAGAGAGTT (SEQ ID NO: 755) |
| 346 | TGFBR2 (SEQ ID NO: 43) | GGTAGTTATGAGAGAGTT (SEQ ID NO: 756) |
| 347 | TIMP3 (SEQ ID NO: 44) | AGGTTTTTCGTTGGAGAA (SEQ ID NO: 757) |
| 348 | TIMP3 (SEQ ID NO: 44) | AGGTTTTTTGTTGGAGAA (SEQ ID NO: 758) |
| 349 | TIMP3 (SEQ ID NO: 44) | GAAAATATCGGTATTTTG (SEQ ID NO: 759) |
| 350 | TIMP3 (SEQ ID NO: 44) | GAAAATATTGGTATTTTG (SEQ ID NO: 760) |
| 351 | TIMP3 (SEQ ID NO: 44) | GGGATAAGCGAATTTTTT (SEQ ID NO: 761) |
| 352 | TIMP3 (SEQ ID NO: 44) | GGGATAAGTGAATTTTTT (SEQ ID NO: 762) |
| 353 | TIMP3 (SEQ ID NO: 44) | TTTTATTACGTATGTTTT (SEQ ID NO: 763) |
| 354 | TIMP3 (SEQ ID NO: 44) | TTTTATTATGTATGTTTT (SEQ ID NO: 764) |
| 355 | TP53 (SEQ ID NO: 45) | AAGTTGAACGTTTAGGTA (SEQ ID NO: 765) |
| 356 | TP53 (SEQ ID NO: 45) | AAGTTGAATGTTTAGGTA (SEQ ID NO: 766) |
| 357 | TP53 (SEQ ID NO: 45) | TTTTGAGTCGGTTTAAAG (SEQ ID NO: 767) |
| 358 | TP53 (SEQ ID NO: 45) | TTTTGAGTTGGTTTAAAG (SEQ ID NO: 768) |
| 359 | TP53 (SEQ ID NO: 45) | TATTTATTCGGTGTTGGG (SEQ ID NO: 769) |
| 360 | TP53 (SEQ ID NO: 45) | TATTTATTTGGTGTTGGG (SEQ ID NO: 770) |
| 361 | TP53 (SEQ ID NO: 45) | TTGGATTTCGAAATATTG (SEQ ID NO: 771) |
| 362 | TP53 (SEQ ID NO: 45) | TTGGATTTTGAAATATTG (SEQ ID NO: 772) |
| 363 | TP73 (SEQ ID NO: 46) | TGATTTAGCGTAGGTTTG (SEQ ID NO: 773) |
| 364 | TP73 (SEQ ID NO: 46) | TGATTTAGTGTAGGTTTG (SEQ ID NO: 774) |
| 365 | TP73 (SEQ ID NO: 46) | TTAGAGTTCGAGTTTATA (SEQ ID NO: 775) |
| 366 | TP73 (SEQ ID NO: 46) | TTAGAGTTTGAGTTTATA (SEQ ID NO: 776) |
| 367 | TP73 (SEQ ID NO: 46) | AAGTTACGGGTTTTATTG (SEQ ID NO: 915) |
| 368 | TP73 (SEQ ID NO: 46) | AAGTTATGGGTTTTATTG (SEQ ID NO: 916) |
| 369 | TP73 (SEQ ID NO: 46) | GGAAGTTTCGATGGTTTA (SEQ ID NO: 777) |
| 370 | TP73 (SEQ ID NO: 46) | GGAAGTTTTGATGGTTTA (SEQ ID NO: 778) |
| 371 | VHL (SEQ ID NO: 47) | TTTATAAGCGTGATGATT (SEQ ID NO: 779) |
| 372 | VHL (SEQ ID NO: 47) | TTTATAAGTGTGATGATT (SEQ ID NO: 780) |
| 373 | VHL (SEQ ID NO: 47) | GGTGTTTTCGTGTGAGAT (SEQ ID NO: 781) |
| 374 | VHL (SEQ ID NO: 47) | GGTGTTTTTGTGTGAGAT (SEQ ID NO: 782) |
| 375 | VHL (SEQ ID NO: 47) | TGTGAGATGCGTTATTTT (SEQ ID NO: 783) |
| 376 | VHL (SEQ ID NO: 47) | TGTGAGATGTGTTATTTT (SEQ ID NO: 784) |
| 377 | VHL (SEQ ID NO: 47) | TATATTGCGCGTTTGATA (SEQ ID NO: 785) |
| 378 | VHL (SEQ ID NO: 47) | TATATTGTGTGTTTGATA (SEQ ID NO: 786) |
| 379 | CDKN1C (SEQ ID NO: 48) | ATGAAGAACGGTTAAGGG (SEQ ID NO: 787) |
| 380 | CDKN1C (SEQ ID NO: 48) | ATGAAGAATGGTTAAGGG (SEQ ID NO: 788) |
| 381 | CDKN1C (SEQ ID NO: 48) | TTTTATTTCGAGTTAGGT (SEQ ID NO: 789) |
| 382 | CDKN1C (SEQ ID NO: 48) | TTTTATTTTGAGTTAGGT (SEQ ID NO: 790) |
| 383 | CDKN1C (SEQ ID NO: 48) | TTAAGTTACGGTTATTAG (SEQ ID NO: 791) |
| 384 | CDKN1C (SEQ ID NO: 48) | TTAAGTTATGGTTATTAG (SEQ ID NO: 792) |
| 385 | CDKN1C (SEQ ID NO: 48) | TTAGTGTTCGTTTGGAAT (SEQ ID NO: 793) |
| 386 | CDKN1C (SEQ ID NO: 48) | TTAGTGTTTGTTTGGAAT (SEQ ID NO: 794) |
| 387 | CAV1 (SEQ ID NO: 49) | TTGGTATCGTTGAAGAAT (SEQ ID NO: 795) |
| 388 | CAV1 (SEQ ID NO: 49) | TTGGTATTGTTGAAGAAT (SEQ ID NO: 796) |
| 389 | CAV1 (SEQ ID NO: 49) | TAGATTCGGAGGTAGGTA (SEQ ID NO: 911) |
| 390 | CAV1 (SEQ ID NO: 49) | TAGATTTGGAGGTAGGTA (SEQ ID NO: 912) |
| 391 | CAV1 (SEQ ID NO: 49) | TGGGGGTTCGAAAAAGTG (SEQ ID NO: 797) |
| 392 | CAV1 (SEQ ID NO: 49) | TGGGGGTTTGAAAAAGTG (SEQ ID NO: 798) |
| 393 | CAV1 (SEQ ID NO: 49) | GAAGTGTTCGTTTTTGTT (SEQ ID NO: 799) |
| 394 | CAV1 (SEQ ID NO: 49) | GAAGTGTTTGTTTTTGTT (SEQ ID NO: 800) |
| 395 | CDH13 (SEQ ID NO: 50) | GAAGTGGTCGTTAGTTTT (SEQ ID NO: 801) |
| 396 | CDH13 (SEQ ID NO: 50) | GAAGTGGTTGTTAGTTTTT (SEQ ID NO: 802) |
| 397 | CDH13 (SEQ ID NO: 50) | TTGTTTAGCGTGATTTGT (SEQ ID NO: 803) |
| 398 | CDH13 (SEQ ID NO: 50) | TTGTTTAGTGTGATTTGT (SEQ ID NO: 804) |
| 399 | CDH13 (SEQ ID NO: 50) | AAGGAATTCGTTTTGTAA (SEQ ID NO: 903) |
| 400 | CDH13 (SEQ ID NO: 50) | AAGGAATTTGTTTTGTAA (SEQ ID NO: 904) |
| 401 | CDH13 (SEQ ID NO: 50) | AATGTTTTCGTGATGTTG (SEQ ID NO: 895) |
| 402 | CDH13 (SEQ ID NO: 50) | AATGTTTTTGTGATGTTG (SEQ ID NO: 896) |
| 403 | DRG1 (SEQ ID NO: 51) | GAGTAGGACGGTGTTAAG (SEQ ID NO: 805) |
| 404 | DRG1 (SEQ ID NO: 51) | GAGTAGGATGGTGTTAAG (SEQ ID NO: 806) |
| 405 | DRG1 (SEQ ID NO: 51) | AAATTTAACGTTGGGTAG (SEQ ID NO: 807) |
| 406 | DRG1 (SEQ ID NO: 51) | AAATTTAATGTTGGGTAG (SEQ ID NO: 808) |
| 407 | DRG1 (SEQ ID NO: 51) | GATAATGACGGTGTTAGT (SEQ ID NO: 809) |
| 408 | DRG1 (SEQ ID NO: 51) | GATAATGATGGTGTTAGT (SEQ ID NO: 810) |
| 409 | DRG1 (SEQ ID NO: 51) | TGGTTGTACGTTAGGAGT (SEQ ID NO: 811) |
| 410 | DRG1 (SEQ ID NO: 51) | TGGTTGTATGTTAGGAGT (SEQ ID NO: 812) |
| 411 | PTGS2 (SEQ ID NO: 52) | TTTATTTTCGTGGGTAAA (SEQ ID NO: 913) |
| 412 | PTGS2 (SEQ ID NO: 52) | TTTATTTTTGTGGGTAAA (SEQ ID NO: 914) |
| 413 | PTGS2 (SEQ ID NO: 52) | AGTTATTTCGTTATATGG (SEQ ID NO: 1007) |
| 414 | PTGS2 (SEQ ID NO: 52) | AGTTATTTTGTTATATGG (SEQ ID NO: 1008) |
| 415 | PTGS2 (SEQ ID NO: 52) | ATTTAAGGCGATTAGTTT (SEQ ID NO: 813) |
| 416 | PTGS2 (SEQ ID NO: 52) | ATTTAAGGTGATTAGTTT (SEQ ID NO: 814) |
| 417 | PTGS2 (SEQ ID NO: 52) | ATATTTGGCGGAAATTTG (SEQ ID NO: 1023) |
| 418 | PTGS2 (SEQ ID NO: 52) | ATATTTGGTGGAAATTTG (SEQ ID NO: 1024) |
| 419 | THBS1 (SEQ ID NO: 53) | GGAGAGTTAGCGAGGGTT (SEQ ID NO: 815) |
| 420 | THBS1 (SEQ ID NO: 53) | GGAGAGTTAGTGAGGGTT (SEQ ID NO: 816) |
| 421 | THBS1 (SEQ ID NO: 53) | TATTTTAACGAATGGTTT (SEQ ID NO: 817) |
| 422 | THBS1 (SEQ ID NO: 53) | TATTTTAATGAATGGTTT (SEQ ID NO: 818) |
| 423 | THBS1 (SEQ ID NO: 53) | TTATAAAACGGGTTTAGT (SEQ ID NO: 819) |
| 424 | THBS1 (SEQ ID NO: 53) | TTATAAAATGGGTTTAGT (SEQ ID NO: 820) |
| 425 | THBS1 (SEQ ID NO: 53) | AGGTATTTCGGGAGATTA (SEQ ID NO: 821) |
| 426 | THBS1 (SEQ ID NO: 53) | AGGTATTTTGGGAGATTA (SEQ ID NO: 822) |
| 427 | TPEF (=TMEFF2; =HPP1) (SEQ ID NO: 54) | ATTTGTTTCGATTAATTT (SEQ ID NO: 979) |
| 428 | TPEF (=TMEFF2;=HPP1) (SEQ ID NO: 54) | ATTTGTTTTGATTAATTT (SEQ ID NO: 980) |
| 429 | TPEF (=TMEFF2;=HPP1) (SEQ ID NO: 54) | ATAGGTTACGGGTTGGAG (SEQ ID NO: 917) |
| 430 | TPEF (=TMEFF2;=HPP1) (SEQ ID NO: 54) | ATAGGTTATGGGTTGGAG (SEQ ID NO: 918) |
| 431 | TPEF (=TMEFF2;=HPP1) (SEQ ID NO: 54) | AATTTGCGAACGTTTGGG (SEQ ID NO: 899) |
| 432 | TPEF (=TMEFF2;=HPP1) (SEQ ID NO: 54) | AATTTGTGAATGTTTGGG (SEQ ID NO: 900) |
| 433 | DNMT1 (SEQ ID NO: 55) | AGTGGGTTCGTTTAAGTT (SEQ ID NO: 823) |
| 434 | DNMT1 (SEQ ID NO: 55) | AGTGGGTTTGTTTAAGTT (SEQ ID NO: 824) |
| 435 | DNMT1 (SEQ ID NO: 55) | TTTTTTACGCGGAGTAGT (SEQ ID NO: 825) |
| 436 | DNMT1 (SEQ ID NO: 55) | TTTTTTATGTGGAGTAGT (SEQ ID NO: 826) |
| 437 | DNMT1 (SEQ ID NO: 55) | GAGAGAGGCGATATTTTG (SEQ ID NO: 827) |
| 438 | DNMT1 (SEQ ID NO: 55) | GAGAGAGGTGATATTTTG (SEQ ID NO: 828) |
| 439 | DNMT1 (SEQ ID NO: 55) | GTATTAAACGGAGAGAGG (SEQ ID NO: 829) |
| 440 | DNMT1 (SEQ ID NO: 55) | GTATTAAATGGAGAGAGG (SEQ ID NO: 830) |
| 441 | CEA (SEQ ID NO: 56) | AAGTGTTCGCGGTTGTTT (SEQ ID NO: 1003) |
| 442 | CEA (SEQ ID NO: 56) | AAGTGTTTGTGGTTGTTT (SEQ ID NO: 1004) |
| 443 | CEA (SEQ ID NO: 56) | TTTTGAGTCGTAGTTTAG (SEQ ID NO: 831) |
| 444 | CEA (SEQ ID NO: 56) | TTTTGAGTTGTAGTTTAG (SEQ ID NO: 832) |
| 445 | CEA (SEQ ID NO: 56) | AATAGATACGGAGAGGGA (SEQ ID NO: 833) |
| 446 | CEA (SEQ ID NO: 56) | AATAGATATGGAGAGGGA (SEQ ID NO: 834) |
| 447 | MB (SEQ ID NO: 57) | AGAAGGTGCGTGAGAGGT (SEQ ID NO: 835) |
| 448 | MB (SEQ ID NO: 57) | AGAAGGTGTGTGAGAGGT (SEQ ID NO: 836) |
| 449 | MB (SEQ ID NO: 57) | GGGTTAGTCGGGGTATTT (SEQ ID NO: 837) |
| 450 | MB (SEQ ID NO: 57) | GGGTTAGTTGGGGTATTT (SEQ ID NO: 838) |
| 451 | MB (SEQ ID NO: 57) | GGGGATAGCGAGTTATTG (SEQ ID NO: 839) |
| 452 | MB (SEQ ID NO: 57) | GGGGATAGTGAGTTATTG (SEQ ID NO: 840) |
| 453 | MB (SEQ ID NO: 57) | TTAGATTGCGTTATGGGG (SEQ ID NO: 841) |
| 454 | MB (SEQ ID NO: 57) | TTAGATTGTGTTATGGGG (SEQ ID NO: 842) |
| 455 | PCNA (SEQ ID NO: 58) | TAAAGAGGCGGGGAGATT (SEQ ID NO: 1013) |
| 456 | PCNA (SEQ ID NO: 58) | TAAAGAGGTGGGGAGATT (SEQ ID NO: 1014) |
| 457 | PCNA (SEQ ID NO: 58) | TATGGATACGATTGGTTT (SEQ ID NO: 843) |
| 458 | PCNA (SEQ ID NO: 58) | TATGGATATGATTGGTTT (SEQ ID NO: 844) |
| 459 | PCNA (SEQ ID NO: 58) | GTATTAAACGGTTGTAGG (SEQ ID NO: 845) |
| 460 | PCNA (SEQ ID NO: 58) | GTATTAAATGGTTGTAGG (SEQ ID NO: 846) |
| 461 | PCNA (SEQ ID NO: 58) | TTTGAAGTCGAAATTAGT (SEQ ID NO: 847) |
| 462 | PCNA (SEQ ID NO: 58) | TTTGAAGTTGAAATTAGT (SEQ ID NO: 848) |
| 463 | CDC2 (SEQ ID NO: 59) | TGGAATTTCGATGTAAAT (SEQ ID NO: 849) |
| 464 | CDC2 (SEQ ID NO: 59) | TGGAATTTTGATGTAAAT (SEQ ID NO: 850) |
| 465 | CDC2 (SEQ ID NO: 59) | TAGTAGGACGATATTTTT (SEQ ID NO: 851) |
| 466 | CDC2 (SEQ ID NO: 59) | TAGTAGGATGATATTTTT (SEQ ID NO: 852) |
| 467 | CDC2 (SEQ ID NO: 59) | TAGTTATTCGGGAAGGTT (SEQ ID NO: 853) |
| 468 | CDC2 (SEQ ID NO: 59) | TAGTTATTTGGGAAGGTT (SEQ ID NO: 854) |
| 469 | CDC2 (SEQ ID NO: 59) | AAATTGTTCGTATTTGGT (SEQ ID NO: 855) |
| 470 | CDC2 (SEQ ID NO: 59) | AAATTGTTTGTATTTGGT (SEQ ID NO: 856) |
| 471 | ESR1 (SEQ ID NO: 60) | AGATATATCGGAGTTTGG (SEQ ID NO: 857) |
| 472 | ESR1 (SEQ ID NO: 60) | AGATATATTGGAGTTTGG (SEQ ID NO: 858) |
| 473 | ESR1 (SEQ ID NO: 60) | GTTTGGTACGGGGTATAT (SEQ ID NO: 859) |
| 474 | ESR1 (SEQ ID NO: 60) | GTTTGGTATGGGGTATAT (SEQ ID NO: 860) |
| 475 | ESR1 (SEQ ID NO: 60) | TTTTAAATCGAGTTGTGT (SEQ ID NO: 861) |
| 476 | ESR1 (SEQ ID NO: 60) | TTTTAAATTGAGTTGTGT (SEQ ID NO: 862) |
| 477 | ESR1 (SEQ ID NO: 60) | TATGAGTTCGGGAGATTA (SEQ ID NO: 863) |
| 478 | ESR1 (SEQ ID NO: 60) | TATGAGTTTGGGAGATTA (SEQ ID NO: 864) |
| 479 | ESR1 (SEQ ID NO: 60) | TGGAGGTTCGGGAGTTTA (SEQ ID NO: 969) |
| 480 | ESR1 (SEQ ID NO: 60) | TGGAGGTTTGGGAGTTTA (SEQ ID NO: 970) |
| 481 | CASP8 (SEQ ID NO: 61) | GAATGAGTCGAGGAAGGT (SEQ ID NO: 865) |
| 482 | CASP8 (SEQ ID NO: 61) | GAATGAGTTGAGGAAGGT (SEQ ID NO: 866) |
| 483 | CASP8 (SEQ ID NO: 61) | TATTGAGACGTTAAGTAA (SEQ ID NO: 867) |
| 484 | CASP8 (SEQ ID NO: 61) | TATTGAGATGTTAAGTAA (SEQ ID NO: 868) |
| 485 | CASP8 (SEQ ID NO: 61) | TAAGGTTACGTAGTTAGT (SEQ ID NO: 869) |
| 486 | CASP8 (SEQ ID NO: 61) | TAAGGTTATGTAGTTAGT (SEQ ID NO: 870) |
| 487 | CASP8 (SEQ ID NO: 61) | GTTAATAGCGGGGATTTT (SEQ ID NO: 871) |
| 488 | CASP8 (SEQ ID NO: 61) | GTTAATAGTGGGGATTTT (SEQ ID NO: 872) |
| 489 | RASSF1 (SEQ ID NO: 62) | GTAGTTTTCGAGAATGTT (SEQ ID NO: 873) |
| 490 | RASSF1 (SEQ ID NO: 62) | GTAGTTTTTGAGAATGTT (SEQ ID NO: 874) |
| 491 | RASSF1 (SEQ ID NO: 62) | TAATTAGAACGTTTTTTG (SEQ ID NO: 875) |
| 492 | RASSF1 (SEQ ID NO: 62) | TAATTAGAATGTTTTTTG (SEQ ID NO: 876) |
| 493 | RASSF1 (SEQ ID NO: 62) | TAGTTTTCGCGTAGAATT (SEQ ID NO: 877) |
| 494 | RASSF1 (SEQ ID NO: 62) | TAGTTTTTGTGTAGAATT (SEQ ID NO: 878) |
| 495 | RASSF1 (SEQ ID NO: 62) | TTTGTAGCGGGTGGAGTA (SEQ ID NO: 995) |
| 496 | RASSF1 (SEQ ID NO: 62) | TTTGTAGTGGGTGGAGTA (SEQ ID NO: 996) |
| 497 | MSH4 (SEQ ID NO: 63) | TTGTTTCGGCGGTTTTTT (SEQ ID NO: 879) |
| 498 | MSH4 (SEQ ID NO: 63) | TTGTTTTGGTGGTTTTTT (SEQ ID NO: 880) |
| 499 | MSH4 (SEQ ID NO: 63) | TTTTGGTACGTTAGGAGT (SEQ ID NO: 881) |
| 500 | MSH4 (SEQ ID NO: 63) | TTTTGGTATGTTAGGAGT (SEQ ID NO: 882) |
| 501 | MSH4 (SEQ ID NO: 63) | TAAATTTTCGGTTAGTTT (SEQ ID NO: 883) |
| 502 | MSH4 (SEQ ID NO: 63) | TAAATTTTTGGTTAGTTT (SEQ ID NO: 884) |
| 503 | MSH4 (SEQ ID NO: 63) | TTAGAGGTCGGTAGTTTA (SEQ ID NO: 885) |
| 504 | MSH4 (SEQ ID NO: 63) | TTAGAGGTTGGTAGTTTA (SEQ ID NO: 886) |
| 505 | MSH5 (SEQ ID NO: 64) | ATGTTTATCGTTTTGAGT (SEQ ID NO: 887) |
| 506 | MSH5 (SEQ ID NO: 64) | ATGTTTATTGTTTTGAGT (SEQ ID NO: 888) |
| 507 | MSH5 (SEQ ID NO: 64) | ATAGTTGTCGAATGTATG (SEQ ID NO: 889) |
| 508 | MSH5 (SEQ ID NO: 64) | ATAGTTGTTGAATGTATG (SEQ ID NO: 890) |
| 509 | MSH5 (SEQ ID NO: 64) | TAGAAGTGCGAAGGGGTA (SEQ ID NO: 891) |
| 510 | MSH5 (SEQ ID NO: 64) | TAGAAGTGTGAAGGGGTA (SEQ ID NO: 892) |
| 511 | MSH5 (SEQ ID NO: 64) | ATGTAATTCGAATGTTTT (SEQ ID NO: 893) |
| 512 | MSH5 (SEQ ID NO: 64) | ATGTAATTTGAATGTTTT (SEQ ID NO: 894) |

**Table 3: Oligonucleotides used in differentiation between colon adenomas or carcinoma tissue and healthy colon tissue.**

| ***No:*** | ***Gene*** | ***Oligo:*** |
|---|---|---|
| 1 | CDH13 (SEQ ID NO: 50) | AATGTTTTCGTGATGTTG (SEQ ID NO: 895) |
| 2 | CDH13 (SEQ ID NO: 50) | AATGTTTTTGTGATGTTG (SEQ ID NO: 896) |
| 3 | CD44 (SEQ ID NO: 20) | TTGTTTAGCGGATTTTAG (SEQ ID NO: 897) |
| 4 | CD44 (SEQ ID NO: 20) | TTGTTTAGTGGATTTTAG (SEQ ID NO: 898) |
| 5 | TPEF (=TMEFF2; =HPP1) (SEQ ID NO: 54) | AATTTGCGAACGTTTGGG (SEQ ID NO: 899) |
| 6 | TPEF (=TMEFF2; =HPP1) (SEQ ID NO: 54) | AATTTGTGAATGTTTGGG (SEQ ID NO: 900) |
| 7 | CSPG2 (SEQ ID NO: 21) | GGGTAACGTCGAATTTAG (SEQ ID NO: 901) |
| 8 | CSPG2 (SEQ ID NO: 21) | GGGTAATGTTGAATTTAG (SEQ ID NO: 902) |
| 9 | CDH13 (SEQ ID NO: 50) | AAGGAATTCGTTTTGTAA (SEQ ID NO: 903) |
| 10 | CDH13 (SEQ ID NO: 50) | AAGGAATTTGTTTTGTAA (SEQ ID NO: 904) |
| 11 | GSTP1 (SEQ ID NO: 25) | GGAGTTCGCGGGATTTTT (SEQ ID NO: 905) |
| 12 | GSTP1 (SEQ ID NO: 25) | GGAGTTTGTGGGATTTTT (SEQ ID NO: 906) |
| 13 | TGFBR2 (SEQ ID NO: 43) | AAAGTTTTCGGAGGGGTT (SEQ ID NO: 907) |
| 14 | TGFBR2 (SEQ ID NO: 43) | AAAGTTTTTGGAGGGGTT (SEQ ID NO: 908) |
| 15 | N33 (SEQ ID NO: 36) | TGTTATTTCGGAGGGTTT (SEQ ID NO: 909) |
| 16 | N33 (SEQ ID NO: 36) | TGTTATTTTGGAGGGTTT (SEQ ID NO: 910) |
| 17 | CAV1 (SEQ ID NO: 49) | TAGATTCGGAGGTAGGTA (SEQ ID NO: 911) |
| 18 | CAV1 (SEQ ID NO: 49) | TAGATTTGGAGGTAGGTA (SEQ ID NO: 912) |
| 19 | PTGS2 (SEQ ID NO: 52) | TTTATTTTCGTGGGTAAA (SEQ ID NO: 913) |
| 20 | PTGS2 (SEQ ID NO: 52) | TTTATTTTTGTGGGTAAA (SEQ ID NO: 914) |
| 21 | TP73 (SEQ ID NO: 46) | AAGTTACGGGTTTTATTG (SEQ ID NO: 915) |
| 22 | TP73 (SEQ ID NO: 46) | AAGTTATGGGTTTTATTG (SEQ ID NO: 916) |
| 23 | TPEF (=TMEFF2; =HPP1) (SEQ ID NO: 54) | ATAGGTTACGGGTTGGAG (SEQ ID NO: 917) |
| 24 | TPEF (=TMEFF2; =HPP1) (SEQ ID NO: 54) | ATAGGTTATGGGTTGGAG (SEQ ID NO: 918) |
| 25 | CD44 (SEQ ID NO: 20) | GTGGGGTTCGGAGGTATA (SEQ ID NO: 919) |
| 26 | CD44 (SEQ ID NO: 20) | GTGGGGTTTGGAGGTATA (SEQ ID NO: 920) |
| 27 | EYA4 (SEQ ID NO: 24) | AAGTAAGTCGTTGTTGTT (SEQ ID NO: 921) |
| 28 | EYA4 (SEQ ID NO: 24) | AAGTAAGTTGTTGTTGTT (SEQ ID NO: 922) |
| 29 | EYA4 (SEQ ID NO: 24) | AGTGTATGCGTAGAAGGT (SEQ ID NO: 923) |
| 30 | EYA4 (SEQ ID NO: 24) | AGTGTATGTGTAGAAGGT (SEQ ID NO: 924) |
| 31 | GTBP/MSH6 (SEQ ID NO: 26) | TTTGTTGGCGGGAAATTT (SEQ ID NO: 925) |
| 32 | GTBP/MSH6 (SEQ ID NO: 26) | TTTGTTGGTGGGAAATTT (SEQ ID NO: 926) |
| 33 | EGR4 (SEQ ID NO: 4) | GGAGTTTTCGGTATATAT (SEQ ID NO: 927) |
| 34 | EGR4 (SEQ ID NO: 4) | GGAGTTTTTGGTATATAT (SEQ ID NO: 928) |
| 35 | CDH1 (SEQ ID NO: 15) | TAGTGGCGTCGGAATTGT (SEQ ID NO: 929) |
| 36 | CDH1 (SEQ ID NO: 15) | TAGTGGTGTTGGAATTGT (SEQ ID NO: 930) |
| 37 | EGFR (SEQ ID NO: 23) | ATTTGGTTCGATTTGGAT (SEQ ID NO: 931) |
| 38 | EGFR (SEQ ID NO: 23) | ATTTGGTTTGATTTGGAT (SEQ ID NO: 932) |
| 39 | LKB1 (SEQ ID NO: 30) | AGGGAGGTCGTTGGTATT (SEQ ID NO: 933) |
| 40 | LKB1 (SEQ ID NO: 30) | AGGGAGGTTGTTGGTATT (SEQ ID NO: 934) |
| 41 | DAPK1 (SEQ ID NO: 22) | TTGTTTTTCGGAAATTTG (SEQ ID NO: 935) |
| 42 | DAPK1 (SEQ ID NO: 22) | TTGTTTTTTGGAAATTTG (SEQ ID NO: 936) |
| 43 | IGF2 (SEQ ID NO: 29) | GATTAGGGCGGGAAATAT (SEQ ID NO: 937) |
| 44 | IGF2 (SEQ ID NO: 29) | GATTAGGGTGGGAAATAT (SEQ ID NO: 938) |
| 45 | HLA-F (SEQ ID NO: 10) | TATTTGGGCGGGTGAGTG (SEQ ID NO: 939) |
| 46 | HLA-F (SEQ ID NO: 10) | TATTTGGGTGGGTGAGTG (SEQ ID NO: 940) |
| 47 | IGF2 (SEQ ID NO: 29) | TTGTATGGTCGAGTTTAT (SEQ ID NO: 941) |
| 48 | IGF2 (SEQ ID NO: 29) | TTGTATGGTTGAGTTTAT (SEQ ID NO: 942) |
| 49 | N33 (SEQ ID NO: 36) | GTTTAGTTAGCGGGTTTT (SEQ ID NO: 943) |
| 50 | N33 (SEQ ID NO: 36) | GTTTAGTTAGTGGGTTTT (SEQ ID NO: 944) |
| 51 | CSPG2 (SEQ ID NO: 21) | AAAAATTCGCGAGTTTAG (SEQ ID NO: 945) |
| 52 | CSPG2 (SEQ ID NO: 21) | AAAAATTTGTGAGTTTAG (SEQ ID NO: 946) |
| 53 | PTEN (SEQ ID NO: 39) | TGATGTGGCGGGATTTTT (SEQ ID NO: 947) |
| 54 | PTEN (SEQ ID NO: 39) | TGATGTGGTGGGATTTTT (SEQ ID NO: 948) |
| 55 | MLH1 (SEQ ID NO: 32) | TTAGGTAGCGGGTAGTAG (SEQ ID NO: 949) |
| 56 | MLH1 (SEQ ID NO: 32) | TTAGGTAGTGGGTAGTAG (SEQ ID NO: 950) |
| 57 | GTBP/MSH6 (SEQ ID NO: 26) | GAGGAATTCGGGTTTTAG (SEQ ID NO: 951) |
| 58 | GTBP/MSH6 (SEQ ID NO: 26) | GAGGAATTTGGGTTTTAG (SEQ ID NO: 952) |
| 59 | CALCA (SEQ ID NO: 14) | ATTAGGTTCGTGTTTTAG (SEQ ID NO: 953) |
| 60 | CALCA (SEQ ID NO: 14) | ATTAGGTTTGTGTTTTAG (SEQ ID NO: 954) |
| 61 | DAPK1 (SEQ ID NO: 22) | GAAGGGAGCGTATTTTAT (SEQ ID NO: 955) |
| 62 | DAPK1 (SEQ ID NO: 22) | GAAGGGAGTGTATTTTAT (SEQ ID NO: 956) |
| 63 | PTEN (SEQ ID NO: 39) | AGAGTTATCGTTTTGTTT (SEQ ID NO: 957) |
| 64 | PTEN (SEQ ID NO: 39) | AGAGTTATTGTTTTGTTT (SEQ ID NO: 958) |
| 65 | WT1 (SEQ ID NO: 9) | TGTTATATCGGTTAGTTG (SEQ ID NO: 959) |
| 66 | WT1 (SEQ ID NO: 9) | TGTTATATTGGTTAGTTG (SEQ ID NO: 960) |
| 67 | EGFR (SEQ ID NO: 23) | TTTGTATTCGGAGTTGGG (SEQ ID NO: 961) |
| 68 | EGFR (SEQ ID NO: 23) | TTTGTATTTGGAGTTGGG (SEQ ID NO: 962) |
| 69 | CSPG2 (SEQ ID NO: 21) | AAGATTTTCGGTTAGTTT (SEQ ID NO: 963) |
| 70 | CSPG2 (SEQ ID NO: 21) | AAGATTTTTGGTTAGTTT (SEQ ID NO: 964) |
| 71 | LKB1 (SEQ ID NO: 30) | TTTAGGTTCGTAAGTTTA (SEQ ID NO: 965) |
| 72 | LKB1 (SEQ ID NO: 30) | TTTAGGTTTGTAAGTTTA (SEQ ID NO: 966) |
| 73 | WT1 (SEQ ID NO: 9) | TATATTGGCGAAGGTTAA (SEQ ID NO: 967) |
| 74 | WT1 (SEQ ID NO: 9) | TATATTGGTGAAGGTTAA (SEQ ID NO: 968) |
| 75 | ESR1 (SEQ ID NO: 60) | TGGAGGTTCGGGAGTTTA (SEQ ID NO: 969) |
| 76 | ESR1 (SEQ ID NO: 60) | TGGAGGTTTGGGAGTTTA (SEQ ID NO: 970) |
| 77 | APC (SEQ ID NO: 12) | TTTAATCGTATAGTTTGT (SEQ ID NO: 971) |
| 78 | APC (SEQ ID NO: 12) | TTTAATTGTATAGTTTGT (SEQ ID NO: 972) |
| 79 | IGF2 (SEQ ID NO: 29) | AGTTTGAACGATGTAAGA (SEQ ID NO: 973) |
| 80 | IGF2 (SEQ ID NO: 29) | AGTTTGAATGATGTAAGA (SEQ ID NO: 974) |
| 81 | MYOD1 (SEQ ID NO: 8) | AATTAGGTCGGATAGGAG (SEQ ID NO: 975) |
| 82 | MYOD1 (SEQ ID NO: 8) | AATTAGGTTGGATAGGAG (SEQ ID NO: 976) |
| 83 | N33 (SEQ ID NO: 36) | TTGGTTCGGGAAAGGTAA (SEQ ID NO: 977) |
| 84 | N33 (SEQ ID NO: 36) | TTGGTTTGGGAAAGGTAA (SEQ ID NO: 978) |
| 85 | TPEF (=TMEFF2; =HPP1) (SEQ ID NO: 54) | ATTTGTTTCGATTAATTT (SEQ ID NO: 979) |
| 86 | TPEF (=TMEFF2; =HPP1) (SEQ ID NO: 54) | ATTTGTTTTGATTAATTT (SEQ ID NO: 980) |
| 87 | CDKN2a (SEQ ID NO: 18) | AATAGTTACGGTCGGAGG (SEQ ID NO: 981) |
| 88 | CDKN2a (SEQ ID NO: 18) | AATAGTTATGGTTGGAGG (SEQ ID NO: 982) |
| 89 | CDH1 (SEQ ID NO: 15) | AGGGTTATCGCGTTTATG (SEQ ID NO: 983) |
| 90 | CDH1 (SEQ ID NO: 15) | AGGGTTATTGTGTTTATG (SEQ ID NO: 984) |
| 91 | APC (SEQ ID NO: 12) | TATTTTGGCGGGTTGTAT (SEQ ID NO: 985) |
| 92 | APC (SEQ ID NO: 12) | TATTTTGGTGGGTTGTAT (SEQ ID NO: 986) |

**Table 4: Oligonucleotides used in differentiation between colon carcinoma tissue and healthy colon tissue.**

| ***No:*** | ***Gene*** | ***Oligo:*** |
|---|---|---|
| 1 | CDH13 (SEQ ID NO: 50) | AATGTTTTCGTGATGTTG (SEQ ID NO: 895) |
| 2 | CDH13 (SEQ ID NO: 50) | AATGTTTTTGTGATGTTG (SEQ ID NO: 896) |
| 3 | TPEF (=TMEFF2; =HPP1) (SEQ ID NO: 54) | AATTTGCGAACGTTTGGG (SEQ ID NO: 899) |
| 4 | TPEF (=TMEFF2; =HPP1) (SEQ ID NO: 54) | AATTTGTGAATGTTTGGG (SEQ ID NO: 900) |
| 5 | CDH13 (SEQ ID NO: 50) | AAGGAATTCGTTTTGTAA (SEQ ID NO: 903) |
| 6 | CDH13 (SEQ ID NO: 50) | AAGGAATTTGTTTTGTAA (SEQ ID NO: 904) |
| 7 | CSPG2 (SEQ ID NO: 21) | GGGTAACGTCGAATTTAG (SEQ ID NO: 901) |
| 8 | CSPG2 (SEQ ID NO: 21) | GGGTAATGTTGAATTTAG (SEQ ID NO: 902) |
| 9 | CD44 (SEQ ID NO: 20) | TTGTTTAGCGGATTTTAG (SEQ ID NO: 897) |
| 10 | CD44 (SEQ ID NO: 20) | TTGTTTAGTGGATTTTAG (SEQ ID NO: 898) |
| 11 | EYA4 (SEQ ID NO: 24) | AGTGTATGCGTAGAAGGT (SEQ ID NO: 923) |
| 12 | EYA4 (SEQ ID NO: 24) | AGTGTATGTGTAGAAGGT (SEQ ID NO: 924) |
| 13 | APC (SEQ ID NO: 12) | TTTAATCGTATAGTTTGT (SEQ ID NO: 971) |
| 14 | APC (SEQ ID NO: 12) | TTTAATTGTATAGTTTGT (SEQ ID NO: 972) |
| 15 | PTGS2 (SEQ ID NO: 52) | TTTATTTTCGTGGGTAAA (SEQ ID NO: 913) |
| 16 | PTGS2 (SEQ ID NO: 52) | TTTATTTTTGTGGGTAAA (SEQ ID NO: 914) |
| 17 | EYA4 (SEQ ID NO: 24) | AAGTAAGTCGTTGTTGTT (SEQ ID NO: 921) |
| 18 | EYA4 (SEQ ID NO: 24) | AAGTAAGTTGTTGTTGTT (SEQ ID NO: 922) |
| 19 | PTEN (SEQ ID NO: 39) | ATTTTGCGTTCGTATTTA (SEQ ID NO: 987) |
| 20 | PTEN (SEQ ID NO: 39) | ATTTTGTGTTTGTATTTA (SEQ ID NO: 988) |
| 21 | GSTP1 (SEQ ID NO: 25) | GGAGTTCGCGGGATTTTT (SEQ ID NO: 905) |
| 22 | GSTP1 (SEQ ID NO: 25) | GGAGTTTGTGGGATTTTT (SEQ ID NO: 906) |
| 23 | CAV1 (SEQ ID NO: 49) | TAGATTCGGAGGTAGGTA (SEQ ID NO: 911) |
| 24 | CAV1 (SEQ ID NO: 49) | TAGATTTGGAGGTAGGTA (SEQ ID NO: 912) |
| 25 | EGFR (SEQ ID NO: 23) | ATTTGGTTCGATTTGGAT (SEQ ID NO: 931) |
| 26 | EGFR (SEQ ID NO: 23) | ATTTGGTTTGATTTGGAT (SEQ ID NO: 932) |
| 27 | N33 (SEQ ID NO: 36) | TGTTATTTCGGAGGGTTT (SEQ ID NO: 909) |
| 28 | N33 (SEQ ID NO: 36) | TGTTATTTTGGAGGGTTT (SEQ ID NO: 910) |
| 29 | IGF2 (SEQ ID NO: 29) | TTGTATGGTCGAGTTTAT (SEQ ID NO: 941) |
| 30 | IGF2 (SEQ ID NO: 29) | TTGTATGGTTGAGTTTAT (SEQ ID NO: 942) |
| 31 | HLA-F (SEQ ID NO: 10) | AGTTGTTTCGTAGATATT (SEQ ID NO: 989) |
| 32 | HLA-F (SEQ ID NO: 10) | AGTTGTTTTGTAGATATT (SEQ ID NO: 990) |
| 33 | TPEF (=TMEFF2; =HPP1) (SEQ ID NO: 54) | ATAGGTTACGGGTTGGAG (SEQ ID NO: 917) |
| 34 | TPEF (=TMEFF2; =HPP1) (SEQ ID NO: 54) | ATAGGTTATGGGTTGGAG (SEQ ID NO: 918) |
| 35 | TP73 (SEQ ID NO: 46) | AAGTTACGGGTTTTATTG (SEQ ID NO: 915) |
| 36 | TP73 (SEQ ID NO: 46) | AAGTTATGGGTTTTATTG (SEQ ID NO: 916) |

**Table 5: Oligonucleotides used in differentiation between colon adenoma tissue and healthy colon tissue.**

| ***No:*** | ***Gene*** | ***Oligo:*** |
|---|---|---|
| 1 | CD44 (SEQ ID NO: 20) | TTGTTTAGCGGATTTTAG (SEQ ID NO: 897) |
| 2 | CD44 (SEQ ID NO: 20) | TTGTTTAGTGGATTTTAG (SEQ ID NO: 898) |
| 3 | HLA-F (SEQ ID NO: 10) | TATTTGGGCGGGTGAGTG (SEQ ID NO: 939) |
| 4 | HLA-F (SEQ ID NO: 10) | TATTTGGGTGGGTGAGTG (SEQ ID NO: 940) |
| 5 | TGFBR2 (SEQ ID NO: 43) | AAAGTTTTCGGAGGGGTT (SEQ ID NO: 907) |
| 6 | TGFBR2 (SEQ ID NO: 43) | AAAGTTTTTGGAGGGGTT (SEQ ID NO: 908) |
| 7 | GTBP/MSH6 (SEQ ID NO: 26) | GAGGAATTCGGGTTTTAG (SEQ ID NO: 951) |
| 8 | GTBP/MSH6 (SEQ ID NO: 26) | GAGGAATTTGGGTTTTAG (SEQ ID NO: 952) |
| 9 | GTBP/MSH6 (SEQ ID NO: 26) | TTTGTTGGCGGGAAATTT (SEQ ID NO: 925) |
| 10 | GTBP/MSH6 (SEQ ID NO: 26) | TTTGTTGGTGGGAAATTT (SEQ ID NO: 926) |
| 11 | LKB1 (SEQ ID NO: 30) | AGGGAGGTCGTTGGTATT (SEQ ID NO: 933) |
| 12 | LKB1 (SEQ ID NO: 30) | AGGGAGGTTGTTGGTATT (SEQ ID NO: 934) |
| 13 | CD44 (SEQ ID NO: 20) | GTGGGGTTCGGAGGTATA (SEQ ID NO: 919) |
| 14 | CD44 (SEQ ID NO: 20) | GTGGGGTTTGGAGGTATA (SEQ ID NO: 920) |
| 15 | N33 (SEQ ID NO: 36) | GTTTAGTTAGCGGGTTTT (SEQ ID NO: 943) |
| 16 | N33 (SEQ ID NO: 36) | GTTTAGTTAGTGGGTTTT (SEQ ID NO: 944) |
| 17 | CDH13 (SEQ ID NO: 50) | AATGTTTTCGTGATGTTG (SEQ ID NO: 895) |
| 18 | CDH13 (SEQ ID NO: 50) | AATGTTTTTGTGATGTTG (SEQ ID NO: 896) |
| 19 | TP73 (SEQ ID NO: 46) | AAGTTACGGGTTTTATTG (SEQ ID NO: 915) |
| 20 | TP73 (SEQ ID NO: 46) | AAGTTATGGGTTTTATTG (SEQ ID NO: 916) |
| 21 | PTEN (SEQ ID NO: 39) | TGATGTGGCGGGATTTTT (SEQ ID NO: 947) |
| 22 | PTEN (SEQ ID NO: 39) | TGATGTGGTGGGATTTTT (SEQ ID NO: 948) |
| 23 | N33 (SEQ ID NO: 36) | TGTTATTTCGGAGGGTTT (SEQ ID NO: 909) |
| 24 | N33 (SEQ ID NO: 36) | TGTTATTTTGGAGGGTTT (SEQ ID NO: 910) |
| 25 | TPEF (=TMEFF2; =HPP1) (SEQ ID NO: 54) | AATTTGCGAACGTTTGGG (SEQ ID NO: 899) |
| 26 | TPEF (=TMEFF2; =HPP1) (SEQ ID NO: 54) | AATTTGTGAATGTTTGGG (SEQ ID NO: 900) |
| 27 | GSTP1 (SEQ ID NO: 25) | GGAGTTCGCGGGATTTTT (SEQ ID NO: 905) |
| 28 | GSTP1 (SEQ ID NO: 25) | GGAGTTTGTGGGATTTTT (SEQ ID NO: 906) |
| 29 | EGFR (SEQ ID NO: 23) | TTTGTATTCGGAGTTGGG (SEQ ID NO: 961) |
| 30 | EGFR (SEQ ID NO: 23) | TTTGTATTTGGAGTTGGG (SEQ ID NO: 962) |
| 31 | RARB (SEQ ID NO: 40) | TAGTAGTTCGGGTAGGGT (SEQ ID NO: 991) |
| 32 | RARB (SEQ ID NO: 40) | TAGTAGTTTGGGTAGGGT (SEQ ID NO: 992) |
| 33 | N33 (SEQ ID NO: 36) | ATTTAGTTCGGGGGAGGA (SEQ ID NO: 993) |
| 34 | N33 (SEQ ID NO: 36) | ATTTAGTTTGGGGGAGGA (SEQ ID NO: 994) |
| 35 | CAV1 (SEQ ID NO: 49) | TAGATTCGGAGGTAGGTA (SEQ ID NO: 911) |
| 36 | CAV1 (SEQ ID NO: 49) | TAGATTTGGAGGTAGGTA (SEQ ID NO: 912) |
| 37 | TPEF (=TMEFF2; =HPP1) (SEQ ID NO: 54) | ATAGGTTACGGGTTGGAG (SEQ ID NO: 917) |
| 38 | TPEF (=TMEFF2; =HPP1) (SEQ ID NO: 54) | ATAGGTTATGGGTTGGAG (SEQ ID NO: 918) |
| 39 | CDKN2a (SEQ ID NO: 18) | AATAGTTACGGTCGGAGG (SEQ ID NO: 981) |
| 40 | CDKN2a (SEQ ID NO: 18) | AATAGTTATGGTTGGAGG (SEQ ID NO: 982) |
| 41 | N33 (SEQ ID NO: 36) | TTGGTTCGGGAAAGGTAA (SEQ ID NO: 977) |
| 42 | N33 (SEQ ID NO: 36) | TTGGTTTGGGAAAGGTAA (SEQ ID NO: 978) |
| 43 | MLH1 (SEQ ID NO: 32) | TTAGGTAGCGGGTAGTAG (SEQ ID NO: 949) |
| 44 | MLH1 (SEQ ID NO: 32) | TTAGGTAGTGGGTAGTAG (SEQ ID NO: 950) |
| 45 | APC (SEQ ID NO: 12) | TATTTTGGCGGGTTGTAT (SEQ ID NO: 985) |
| 46 | APC (SEQ ID NO: 12) | TATTTTGGTGGGTTGTAT (SEQ ID NO: 986) |
| 47 | CSPG2 (SEQ ID NO: 21) | GGGTAACGTCGAATTTAG (SEQ ID NO: 901) |
| 48 | CSPG2 (SEQ ID NO: 21) | GGGTAATGTTGAATTTAG (SEQ ID NO: 902) |
| 49 | CDH1 (SEQ ID NO: 15) | TAGTGGCGTCGGAATTGT (SEQ ID NO: 929) |
| 50 | CDH1 (SEQ ID NO: 15) | TAGTGGTGTTGGAATTGT (SEQ ID NO: 930) |
| 51 | PTGS2 (SEQ ID NO: 52) | TTTATTTTCGTGGGTAAA (SEQ ID NO: 913) |
| 52 | PTGS2 (SEQ ID NO: 52) | TTTATTTTTGTGGGTAAA (SEQ ID NO: 914) |
| 53 | RASSF1 (SEQ ID NO: 62) | TTTGTAGCGGGTGGAGTA (SEQ ID NO: 995) |
| 54 | RASSF1 (SEQ ID NO: 62) | TTTGTAGTGGGTGGAGTA (SEQ ID NO: 996) |
| 55 | WT1 (SEQ ID NO: 9) | TATATTGGCGAAGGTTAA (SEQ ID NO: 967) |
| 56 | WT1 (SEQ ID NO: 9) | TATATTGGTGAAGGTTAA (SEQ ID NO: 968) |
| 57 | CDKN2a (SEQ ID NO: 18) | GGAGTTTTCGGTTGATTG (SEQ ID NO: 997) |
| 58 | CDKN2a (SEQ ID NO: 18) | GGAGTTTTTGGTTGATTG (SEQ ID NO: 998) |
| 59 | ESR1 (SEQ ID NO: 60) | TGGAGGTTCGGGAGTTTA (SEQ ID NO: 969) |
| 60 | ESR1 (SEQ ID NO: 60) | TGGAGGTTTGGGAGTTTA (SEQ ID NO: 970) |
| 61 | IGF2 (SEQ ID NO: 29) | GATTAGGGCGGGAAATAT (SEQ ID NO: 937) |
| 62 | IGF2 (SEQ ID NO: 29) | GATTAGGGTGGGAAATAT (SEQ ID NO: 938) |
| 63 | MYOD1 (SEQ ID NO: 8) | AATTAGGTCGGATAGGAG (SEQ ID NO: 975) |
| 64 | MYOD1 (SEQ ID NO: 8) | AATTAGGTTGGATAGGAG (SEQ ID NO: 976) |
| 65 | CDH13 (SEQ ID NO: 50) | AAGGAATTCGTTTTGTAA (SEQ ID NO: 903) |
| 66 | CDH13 (SEQ ID NO: 50) | AAGGAATTTGTTTTGTAA (SEQ ID NO: 904) |
| 67 | EGR4 (SEQ ID NO: 4) | GGAGTTTTCGGTATATAT (SEQ ID NO: 927) |
| 68 | EGR4 (SEQ ID NO: 4) | GGAGTTTTTGGTATATAT (SEQ ID NO: 928) |
| 69 | S100A2 (SEQ ID NO: 42) | TATGTATACGAGTATTGG (SEQ ID NO: 999) |
| 70 | S100A2 (SEQ ID NO: 42) | TATGTATATGAGTATTGG (SEQ ID NO: 1000) |
| 71 | DAPK1 (SEQ ID NO: 22) | TTGTTTTTCGGAAATTTG (SEQ ID NO: 935) |
| 72 | DAPK1 (SEQ ID NO: 22) | TTGTTTTTTGGAAATTTG (SEQ ID NO: 936) |
| 73 | MGMT (SEQ ID NO: 31) | AGTAGGATCGGGATTTTT (SEQ ID NO: 1001) |
| 74 | MGMT (SEQ ID NO: 31) | AGTAGGATTGGGATTTTT (SEQ ID NO: 1002) |
| 75 | EYA4 (SEQ ID NO: 24) | AAGTAAGTCGTTGTTGTT (SEQ ID NO: 921) |
| 76 | EYA4 (SEQ ID NO: 24) | AAGTAAGTTGTTGTTGTT (SEQ ID NO: 922) |
| 77 | CEA (SEQ ID NO: 56) | AAGTGTTCGCGGTTGTTT (SEQ ID NO: 1003) |
| 78 | CEA (SEQ ID NO: 56) | AAGTGTTTGTGGTTGTTT (SEQ ID NO: 1004) |
| 79 | WT1 (SEQ ID NO: 9) | TGTTATATCGGTTAGTTG (SEQ ID NO: 959) |
| 80 | WT1 (SEQ ID NO: 9) | TGTTATATTGGTTAGTTG (SEQ ID NO: 960) |
| 81 | GPIb beta (SEQ ID NO: 7) | GGAGTTCGGTCGGGTTTT (SEQ ID NO: 1005) |
| 82 | GPIb beta (SEQ ID NO: 7) | GGAGTTTGGTTGGGTTTT (SEQ ID NO: 1006) |
| 83 | CALCA (SEQ ID NO: 14) | ATTAGGTTCGTGTTTTAG (SEQ ID NO: 953) |
| 84 | CALCA (SEQ ID NO: 14) | ATTAGGTTTGTGTTTTAG (SEQ ID NO: 954) |
| 85 | PTGS2 (SEQ ID NO: 52) | AGTTATTTCGTTATATGG (SEQ ID NO: 1007) |
| 86 | PTGS2 (SEQ ID NO: 52) | AGTTATTTTGTTATATGG (SEQ ID NO: 1008) |
| 87 | MYOD1 (SEQ ID NO: 8) | GTGTTAGTCGTTTAGGGT (SEQ ID NO: 1009) |
| 88 | MYOD1 (SEQ ID NO: 8) | GTGTTAGTTGTTTAGGGT (SEQ ID NO: 1010) |
| 89 | EYA4 (SEQ ID NO: 24) | AGTGTATGCGTAGAAGGT (SEQ ID NO: 923) |
| 90 | EYA4 (SEQ ID NO: 24) | AGTGTATGTGTAGAAGGT (SEQ ID NO: 924) |
| 91 | CSPG2 (SEQ ID NO: 21) | AAAAATTCGCGAGTTTAG (SEQ ID NO: 945) |
| 92 | CSPG2 (SEQ ID NO: 21) | AAAAATTTGTGAGTTTAG (SEQ ID NO: 946) |
| 93 | PGR (SEQ ID NO: 38) | TTAAGTGTCGGATTTGTG (SEQ ID NO: 1011) |
| 94 | PGR (SEQ ID NO: 38) | TTAAGTGTTGGATTTGTG (SEQ ID NO: 1012) |
| 95 | PCNA (SEQ ID NO: 58) | TAAAGAGGCGGGGAGATT (SEQ ID NO: 1013) |
| 96 | PCNA (SEQ ID NO: 58) | TAAAGAGGTGGGGAGATT (SEQ ID NO: 1014) |
| 97 | MSH3 (SEQ ID NO: 34) | AGTATTTTCGTTTGAGGA (SEQ ID NO: 1015) |
| 98 | MSH3 (SEQ ID NO: 34) | AGTATTTTTGTTTGAGGA (SEQ ID NO: 1016) |
| 99 | WT1 (SEQ ID NO: 9) | TAGTGAGACGAGGTTTTT (SEQ ID NO: 1017) |
| 100 | WT1 (SEQ ID NO: 9) | TAGTGAGATGAGGTTTTT (SEQ ID NO: 1018) |
| 101 | MYC (SEQ ID NO: 35) | TTATAATGCGAGGGTTTG (SEQ ID NO: 1019) |
| 102 | MYC (SEQ ID NO: 35) | TTATAATGTGAGGGTTTG (SEQ ID NO: 1020) |
| 103 | HIC-1 (SEQ ID NO: 27) | TTTTAGAGCGTTAGGGTT (SEQ ID NO: 1021) |
| 104 | HIC-1 (SEQ ID NO: 27) | TTTTAGAGTGTTAGGGTT (SEQ ID NO: 1022) |
| 105 | PTGS2 (SEQ ID NO: 52) | ATATTTGGCGGAAATTTG (SEQ ID NO: 1023) |
| 106 | PTGS2 (SEQ ID NO: 52) | ATATTTGGTGGAAATTTG (SEQ ID NO: 1024) |
| 107 | EGFR (SEQ ID NO: 23) | ATTTGGTTCGATTTGGAT (SEQ ID NO: 931) |
| 108 | EGFR (SEQ ID NO: 23) | ATTTGGTTTGATTTGGAT (SEQ ID NO: 932) |
| 109 | LKB1 (SEQ ID NO: 30) | TTTAGGTTCGTAAGTTTA (SEQ ID NO: 965) |
| 110 | LKB1 (SEQ ID NO: 30) | TTTAGGTTTGTAAGTTTA (SEQ ID NO: 966) |
| 111 | IGF2 (SEQ ID NO: 29) | TTGTATGGTCGAGTTTAT (SEQ ID NO: 941) |
| 112 | IGF2 (SEQ ID NO: 29) | TTGTATGGTTGAGTTTAT (SEQ ID NO: 942) |
| 113 | PTEN (SEQ ID NO: 39) | AGAGTTATCGTTTTGTTT (SEQ ID NO: 957) |
| 114 | PTEN (SEQ ID NO: 39) | AGAGTTATTGTTTTGTTT (SEQ ID NO: 958) |
| 115 | BCL2 (SEQ ID NO: 13) | TTTTGTTACGGTGGTGGA (SEQ ID NO: 1025) |
| 116 | BCL2 (SEQ ID NO: 13) | TTTTGTTATGGTGGTGGA (SEQ ID NO: 1026) |
| 117 | AR (SEQ ID NO: 5) | AGAGGTTGCGTTTTAGAG (SEQ ID NO: 1027) |
| 118 | AR (SEQ ID NO: 5) | AGAGGTTGTGTTTTAGAG (SEQ ID NO: 1028) |
| 119 | CDH1 (SEQ ID NO: 15) | AGGGTTATCGCGTTTATG (SEQ ID NO: 983) |
| 120 | CDH1 (SEQ ID NO: 15) | AGGGTTATTGTGTTTATG (SEQ ID NO: 984) |

**Table 6: Oligonucleotides used in differentiation between colon carcinoma tissue and colon adenoma tissue.**

| ***No:*** | ***Gene*** | ***Oligo:*** |
|---|---|---|
| 1 | CDKN2a (SEQ ID NO: 18) | GTTGTTTTCGGTTGGTGT (SEQ ID NO: 1029) |
| 2 | CDKN2a (SEQ ID NO: 18) | GTTGTTTTTGGTTGGTGT (SEQ ID NO: 1030) |
| 3 | GPIb beta (SEQ ID NO: 7) | GGAGTTCGGTCGGGTTTT (SEQ ID NO: 1005) |
| 4 | GPIb beta (SEQ ID NO: 7) | GGAGTTTGGTTGGGTTTT (SEQ ID NO: 1006) |

**Table 7: Crossreference table to relate numbers used in figure labelling to ID numbers used throughout the document**

| Number in Figures | Gene name |
|---|---|
| Healthy vs Non-Healthy | |
| 50-D | CDH13 |
| 20-C | CD44 |
| 54-C | TPEF (=TMEFF2; =HPP1) |
| 21-C | CSPG2 |
| 50-C | CDH13 |
| 25-B | GSTP1 |
| 43-C | TGFBR2 |
| 36-B | N33 |
| 49-A | CAV1 |
| 52-C | PTGS2 |
| 46-A | TP73 |
| 54-B | TPEF (=TMEFF2; =HPP1) |
| 20-A | CD44 |
| 24-D | EYA4 |
| 24-B | EYA4 |
| 26-B | GTBP/MSH6 |
| 4-C | EGR4 |
| 15-E | CDH1 |
| 23-E | EGFR |
| 30-B | LKB1 |
| 22-D | DAPK1 |
| 29-D | IGF2 |
| 10-A | HLA-F |
| 29-C | IGF2 |
| 36-C | N33 |
| 21-D | CSPG2 |
| 39-D | PTEN |
| 32-B | MLH1 |
| 26-A | GTBP/MSH6 |
| 14-C | CALCA |
| 22-C | DAPK1 |
| 39-C | PTEN |
| 9-D | WT1 |
| 23-A | EGFR |
| 21-A | CSPG2 |
| 30-A | LKB1 |
| 9-C | WT1 |
| 60-E | ESR1 |
| 12-A | APC |
| 29-A | IGF2 |
| 8-D | MYOD1 |
| 36-A | N33 |
| 54-A | TPEF (=TMEFF2; =HPP1) |
| 18-E | CDKN2a |
| 15-D | CDH1 |
| 12-C | APC |

| Healthy vs Carcinoma | |
|---|---|
| 50-D | CDH13 |
| 54-C | TPEF (=TMEFF2; =HPP1) |
| 50-C | CDH13 |
| 21-C | CSPG2 |
| 20-C | CD44 |
| 24-B | EYA4 |
| 12-A | APC |
| 52-C | PTGS2 |
| 24-D | EYA4 |
| 39-B | PGR |
| 25-B | GSTP1 |
| 49-A | CAV1 |
| 23-E | EGFR |
| 36-B | N33 |
| 29-C | IGF2 |
| 10-D | HLA-F |
| 54-B | TPEF (=TMEFF2; =HPP1) |
| 46-A | TP73 |

| Healthy vs Adenoma | |
|---|---|
| 20-C | CD44 |
| 10-A | HLA-F |
| 43-C | TGFBR2 |
| 26-A | GTBP/MSH6 |
| 26-B | GTBP/MSH6 |
| 30-B | LKB1 |
| 20-A | CD44 |
| 36-C | N33 |
| 50-D | CDH13 |
| 46-A | TP73 |
| 39-D | PTEN |
| 36-B | N33 |
| 54-C | TPEF (=TMEFF2; =HPP1) |
| 25-B | GSTP1 |
| 23-A | EGFR |
| 40-A | RARB |
| 36-D | N33 |
| 49-A | CAV1 |
| 54-B | TPEF (=TMEFF2; =HPP1) |
| 18-E | CDKN2a |
| 36-A | N33 |
| 32-B | MLH1 |
| 12-C | APC |
| 21-C | CSPG2 |
| 15-E | CDH1 |
| 52-C | PTGS2 |
| 62-D | RASSF1 |
| 9-C | WT1 |
| 18-D | CDKN2a |
| 60-E | ESR1 |
| 29-D | IGF2 |
| 8-D | MYOD1 |
| 50-C | CDH13 |
| 4-C | EGR4 |
| 42-C | S100A2 |
| 22-D | DAPK1 |
| 31-E | MGMT |
| 24-D | EYA4 |
| 56-A | CEA |
| 9-D | WT1 |
| 7-E | GPIb beta |
| 14-C | CALCA |
| 52-D | PTGS2 |
| 8-B | MYOD1 |
| 24-B | EYA4 |
| 21-D | CSPG2 |
| 38-C | PGR |
| 58-A | PCNA |
| 34-D | MSH3 |
| 9-B | WT1 |
| 35-B | MYC |
| 27-C | HIC-1 |
| 52-B | PTGS2 |
| 23-E | EGFR |
| 30-A | LKB1 |
| 29-C | IGF2 |
| 39-C | PTEN |
| 13-D | BCL2 |
| 5-B | AR |
| 15-D | CDH1 |

| Carcinoma vs Adenoma | |
|---|---|
| 18-B | CDKN2a IGPIb |
| 7-E | beta |

## Claims

1. A method for detecting and differentiating between colon cell proliferative disorders, comprising the following steps;
a) providing a body fluid sample containing genomic DNA,
b) extracting said genomic DNA,
c) converting cytosine bases in said genomic DNA sample which are unmethylated at the 5-position, by treatment, to uracil or another base which is dissimilar to cytosine in terms of base pairing behaviour, and
d) identifying the methylation status of one or more cytosine positions of the gene TPEF and/or its regulatory regions.

2. The method according to claim 1, **characterised in that** the reagent is a solution of bisulfite, hydrogen sulfite or disulfite.

3. The method according to claim 1 or 2, **characterised in that** the fragments of said pretreated genomic DNA are amplified by means of the polymerase chain reaction (PCR) prior to step d).

4. The method according to any of claims 1 to 3, **characterised in that** more than ten different fragments having a length of 100 - 2000 base pairs are amplified.

5. The method according to any of claims 1 to 4, **characterised in that** the amplification step is carried out using at least two oligonucleotides whose sequences are each reverse complementary or identical to an at least 18 base-pair long segment of a sequence selected from the group consisting of SEQ ID NO: 239, 240, 367, and 368.

6. The method according to any of claims 1 to 5, **characterised in that** the amplification step preferentially amplifies DNA which is of particular interest in healthy and/or diseased colon tissues, based on the specific genomic methylation status of colon tissue, as opposed to background DNA.

7. The method according to any of claims 1 to 6, **characterised in that** identifying the methylation status of one or more cytosine positions based on said array involves a hybridisation of each amplificates to an oligonucleotide or peptide nucleic acid (PNA)-oligomer.

8. The method according to claim 7, **characterised in that** the amplificates are labelled.

9. The method according to claim 8, further comprising detecting the amplificates or fragments of the amplificates by mass spectrometry.

10. A method for detecting and differentiating between colon cell proliferative disorders, comprising the following steps;
a) providing a body fluid sample containing genomic DNA,
b) extracting said genomic DNA,
c) digesting the genomic DNA of the gene TPEF using a methylation sensitive restriction enzyme, and
d) detecting the DNA fragments generated in the digest of step d).

11. The method according to claim 10, wherein the DNA digest is amplified prior to Step d).

12. The method according to claim 10, wherein the amplification is carried out by means of the polymerase chain reaction (PCR),

13. The method according to claim 11 or 12, wherein the amplification of more than one DNA fragments is carried out in one reaction vessel,

14. The method according to any of claims I to 13, wherein said method differentiates between normal colon tissue and colon cell proliferative disorder tissue, differentiates between colon adenoma tissue and normal colon tissue, differentiates between colon carcinoma tissue and normal colon tissue or differentiates between colon adenoma tissue and colon carcinoma tissue.

15. Use of a gene panel comprising at least one target nucleic acid selected from the gene TPEF (=TMEFF2; =HPP1) and/or its regulatory regions for the detection of colon cell proliferative disorders in body fluids in a method according to any of the preceding claims.

16. Use of a gene panel according to claim 15, wherein said target nucleic acids are selected from the group comprising SEQ ID NO: 239, 240, 367, and 368, and sequences complementary thereto.

17. Use of a gene panel according to claim 15 or 16, wherein said panel is present in the form of a nucleic acid or peptide nucleic acid array for the analysis of colon cell proliferative disorders associated with the methylation state of said target nucleic acids.

18. Use according to claim 17, **characterised in that** a solid phase surface of said array is composed of silicon, glass, polystyrene, aluminium, steel, iron, copper, nickel, silver, or gold.

19. Use of an oligonucleotide containing at least one base sequence having a length of at least 10 nucleotides which hybridises to a nucleic acid selected from the group consisting of SEQ ID NO: 239, 240, 367, and 368 in a method according to claim 1.

20. Use of an oligonucleotide at least 18 bases in length which hybridises to a nucleic acid selected from the group consisting of SEQ ID NO: 239, 240, 367, and 368 in a method according to claim 1.

21. Use of an oligonucleotide according to either of claims 19 or 20 wherein the base sequence thereof comprises at least one CG or TG dinucleotide.

## Patentansprüche

1. Verfahren zum Nachweis und der Differenzierung zwischen proliferativen Störungen von Darmzellen, umfassend die folgenden Schritte;
a) zur Verfügung stellen einer Probe an Körperflüssigkeit enthaltend genomische DNA,
b) Extrahieren der genomischen DNA,
c) Umwandeln von Cytosinbasen in der genomischen DNA Probe, die an der 5-Position nicht methyliert sind, durch Behandlung zu Uracil oder einer anderen Base, die im Hinblick auf ihr Basenpaarungsverhalten von Cytosin unterschiedlich ist, und
d) Identifizieren des Methylierungsstatus einer oder mehrerer Cytosinpositionen des Gens TPEF und/oder seiner regulatorischen Regionen.

2. Verfahren nach Anspruch 1, **dadurch gekennzeichnet, dass** das Reagenz eine Lösung von Bisulfit, Hydrogensulfit oder Disulfit ist.

3. Verfahren nach Anspruch 1 oder 2, **dadurch gekennzeichnet, dass** die Fragmente der vorbehandelten genomischen DNA mittels der Polymerasekettenreaktion (PCR) vor Schritt d) amplifiziert werden.

4. Verfahren nach einem der Ansprüche 1 bis 3, **dadurch gekennzeichnet, dass** mehr als zehn verschiedene Fragmente mit einer Länge von 100 - 2000 Basenpaaren amplifiziert werden.

5. Verfahren nach einem der Ansprüche 1 bis 4, **dadurch gekennzeichnet, dass** der Amplifikationsschritt unter der Verwendung von mindestens zwei Oligonukleotiden durchgeführt wird, deren Sequenzen jeweils revers komplementär oder identisch zu einem mindestens 18 Basenpaaren langen Segment einer Sequenz ausgewählt aus der Gruppe bestehend aus SEQ ID NO: 239, 240, 367 und 368 ist.

6. Verfahren nach einem der Ansprüche 1 bis 5, **dadurch gekennzeichnet, dass** der Amplifikationsschritt bevorzugterweise DNA amplifiziert, die von besonderem Interesse in gesunden und/oder erkrankten Darmgeweben ist, basierend auf dem spezifischen genomischen Methylierungsstatus von Darmgewebe, im Gegensatz zu Hintergrund-DNA.

7. Verfahren nach einem der Ansprüche 1 bis 6, **dadurch gekennzeichnet, dass** das Identifizieren des Methylierungsstatus einer oder mehrerer Cytosinpositionen basierend auf dem Array eine Hybridisierung eines jeden Amplifikats an ein Oligonukleotid oder Peptidnukleinsäure (PNA)-Oligomer einschließt.

8. Verfahren nach Anspruch 7, **dadurch gekennzeichnet, dass** die Amplifikate markiert sind.

9. Verfahren nach Anspruch 8, weiter umfassend Nachweisen der Amplifikate oder Fragmente der Amplifikate mittels Massenspektrometrie.

10. Verfahren zum Nachweis und der Differenzierung zwischen proliferativen Störungen von Darmzellen, umfassend die folgenden Schritte;
a) zur Verfügung stellen einer Probe an Körperflüssigkeit enthaltend genomische DNA,
b) Extrahieren der genomischen DNA,
c) Verdauen der genomischen DNA des Gens TPEF unter der Verwendung eines methylierungssensitiven Restriktionsenzyms, und
d) Nachweisen der in dem Verdau von Schritt d) erzeugten DNA Fragmente.

11. Verfahren nach Anspruch 10, wobei der DNA Verdau vor Schritt d) amplifiziert wird.

12. Verfahren nach Anspruch 10, wobei die Amplifikation mittels der Polymerasekettenreaktion (PCR) durchgeführt wird.

13. Verfahren nach Anspruch 11 oder 12, wobei die Amplifikation von mehr als einem DNA Fragmente in einem Reaktionsgefäß durchgeführt wird.

14. Verfahren nach einem der Ansprüche 1 bis 13, wobei das Verfahren zwischen normalem Darmgewebe und Darmgewebe mit proliferativen Störungen unterscheidet, zwischen Darmadenomgewebe und normalem Darmgewebe unterscheidet, zwischen Darmkarzinomgewebe und normalem Darmgewebe unterscheidet oder zwischen Darmadenomgewebe und Darmkarzinomgewebe unterscheidet.

15. Verwendung eines Genpanels umfassend mindestens eine Zielnukleinsäure ausgewählt aus dem Gen TPEF (=TMEFF2; =HPP1) und/oder seiner regulatorischen Regionen zum Nachweis proliferativen Störungen von Darmzellen in Körperflüssigkeiten in einem Verfahren nach einem der voranstehenden Ansprüche.

16. Verwendung eines Genpanels nach Anspruch 15, wobei die Zielnukleinsäuren ausgewählt sind aus der Gruppe umfassend SEQ ID NO: 239, 240, 367 und 368 und dazu komplementären Sequenzen.

17. Verwendung eines Genpanels nach Anspruch15 oder 16, wobei das Panel in Form eines Nukleinsäure- oder Peptidnukleinsäurearrays für die Analyse von proliferativen Störungen von Darmzellen, die mit dem Methylierungsstatus der Zielnukleinsäure assoziiert sind, vorliegt.

18. Verwendung nach Anspruch 17, **dadurch gekennzeichnet, dass** eine feste Phasenoberfläche des Arrays aus Silizium, Glas, Polystyrol, Aluminium, Stahl, Eisen, Kupfer, Nickel, Silber oder Gold zusammengesetzt ist.

19. Verwendung eines Oligonukleotids enthaltend mindestens eine Basensequenz mit einer Länge von mindestens 10 Nukleotiden, das an eine Nukleinsäure ausgewählt aus der Gruppe bestehend aus SEQ ID NO: 239, 240, 367 und 368 hybridisiert, in einem Verfahren nach Anspruch 1.

20. Verwendung eines Oligonukleotids mit einer Länge von mindestens 18 Basen, das an eine Nukleinsäure ausgewählt aus der Gruppe bestehend aus SEQ ID NO: 239, 240, 367 und 368 hybridisiert, in einem Verfahren nach Anspruch 1.

21. Verwendung eines Oligonukleotids nach entweder Anspruch 1 oder 2, wobei die Basensequenz davon mindestens ein CG oder TG Dinukleotid umfasst.

## Revendications

1. Procédé pour détecter et différencier des troubles prolifératifs des cellules du côlon, comprenant les étapes suivantes :
a) prévoir un échantillon de fluide corporel contenant de l'ADN génomique,
b) extraire ledit ADN génomique,
c) convertir des bases de type cytosine dans ledit échantillon d'ADN génomique, qui sont non méthylées en position 5, par traitement, en uracile ou une autre base différente de la cytosine en termes de comportement d'appariement des bases, et
identifier l'état de méthylation d'une ou plusieurs positions de cytosine du gène TPEF et/ou ses régions régulatoires.

2. Procédé selon la revendication 1, **caractérisé en ce que** le réactif est une solution de bisulfite, sulfite d'hydrogène ou disulfite.

3. Procédé selon la revendication 1 ou 2, **caractérisé en ce que** les fragments dudit ADN génomique prétraité sont amplifiés au moyen de la réaction en chaîne par polymérase (PCR) avant l'étape d).

4. Procédé selon l'une quelconque des revendications 1 à 3, **caractérisé en ce que** plus de dix fragments différents ayant une longueur de 100-2000 paires de bases sont amplifiés.

5. Procédé selon l'une quelconque des revendications 1 à 4, **caractérisé en ce que** l'étape d'amplification est réalisée en utilisant au moins deux oligonucléotides dont les séquences sont chacune inversement complémentaires ou identiques à un segment d'une longueur d'au moins 18 paires de bases d'une séquence choisie dans le groupe comprenant les séquences SEQ ID N° : 239, 240, 367 et 368.

6. Procédé selon l'une quelconque des revendications 1 à 5, **caractérisé en ce que** l'étape d'amplification amplifie, de préférence, l'ADN qui présente un intérêt particulier dans des tissus du côlon sains et/ou malades, sur la base de l'état de méthylation génomique spécifique du tissu du côlon, par opposition à l'ADN de contrôle.

7. Procédé selon l'une quelconque des revendications 1 à 6, **caractérisé en ce que** l'identification de l'état de méthylation d'une ou plusieurs positions de cytosine sur la base de ladite matrice implique une hybridation de chaque amplificat avec un oligonucléotide ou oligomère d'acide nucléique peptidique (PNA).

8. Procédé selon la revendication 7, **caractérisé en ce que** les amplificats sont marqués.

9. Procédé selon la revendication 8, consistant, en outre, à détecter les amplificats ou fragments des amplificats par spectrométrie de masse.

10. Procédé pour détecter et différencier des troubles prolifératifs des cellules du côlon, comprenant les étapes suivantes :
a) prévoir un échantillon de fluide corporel contenant de l'ADN génomique,
b) extraire ledit ADN génomique,
c) digérer l'ADN génomique du gène TPEF au moyen d'une enzyme de restriction sensible à la méthylation, et
d) détecter les fragments d'ADN générés dans le digest de l'étape d).

11. Procédé selon la revendication 10, dans lequel le digest d'ADN est amplifié avant l'étape d).

12. Procédé selon la revendication 10, dans lequel l'amplification est réalisée au moyen de la réaction en chaîne par polymérase (PCR).

13. Procédé selon la revendication 11 ou 12, dans lequel l'amplification de plus d'un fragment d'ADN est réalisée dans une cuve à réaction.

14. Procédé selon l'une quelconque des revendications 1 à 13, **caractérisé en ce que** ledit procédé différencie le tissu normal du côlon du tissu atteint de troubles prolifératifs des cellules du côlon, différencie le tissu d'un côlon atteint d'un adénome du tissu normal du côlon, différencie le tissu d'un côlon atteint d'un carcinome d'un tissu normal du côlon ou différencie le tissu d'un côlon atteint d'un adénome du tissu d'un côlon atteint d'un carcinome.

15. Utilisation d'un panel de gènes comprenant au moins un acide nucléique cible sélectionné à partir du gène TPEF(=TMEFF2 ; =HPP1) et/ou ses régions régulatoires pour la détection de troubles prolifératifs de cellules du côlon dans des fluides corporels dans un procédé selon l'une quelconque des revendications précédentes.

16. Utilisation d'un panel de gènes selon la revendication 15, lesdits acides nucléiques cibles étant choisis dans le groupe comprenant les séquences SEQ ID NO : 239, 240, 367 et 368 et des séquences complémentaires de celles-ci.

17. Utilisation d'un panel de gènes selon la revendication 15 ou 16, ledit panel étant présent sous la forme d'une matrice d'acides nucléiques ou d'acides nucléiques peptidiques pour l'analyse de troubles prolifératifs de cellules du côlon associés à l'état de méthylation desdits acides nucléiques cibles.

18. Utilisation selon la revendication 17, **caractérisée en ce qu'**une surface d'une phase solide de ladite matrice se compose de silicone, verre, polystyrène, aluminium, acier, fonte, cuivre, nickel, argent ou or.

19. Utilisation d'un oligonucléotide contenant au moins un séquence de base d'une longueur d'au moins 10 nucléotides, qui s'hybride à un acide nucléique choisi dans le groupe comprenant les séquences SEQ ID N° : 239, 240, 367 et 368 dans un procédé selon la revendication 1.

20. Utilisation d'un oligonucléotide d'une longueur d'au moins 18 bases, qui s'hybride à un acide nucléique choisi dans le groupe comprenant les séquences SEQ ID N° : 239, 240, 367 et 368 dans un procédé selon la revendication 1.

21. Utilisation d'un oligonucléotide selon l'une quelconque des revendications 13 ou 20, la séquence de base de celui-ci comprenant au moins un dinucléotide CG ou TG.
